# EUROPEAN PATENT APPLICATION

(11) **EP 4 461 329 A1**
(43) Date of publication of application: **13.11.2024**
(21) Application number: 24166896.1
(22) Date of filing: 27.03.2024
(51) Int. Cl.: A61M 5/145

(54) **A SYRINGE POSITIONING ASSEMBLY AND A SYRINGE PUMP**

(30) Priority: 12.05.2023 CN 202310544140; 29.06.2023 CN 202310794612; 11.03.2024 CN 202420465096 U
(71) Applicant: Medcaptain Medical Technology Co., Ltd., Shenzhen, Guangdong 518000 (CN)
(72) Inventor: Liu, Fulin, Shenzhen, 518000 (CN); Huang, Haoke, Shenzhen, 518000 (CN); Dong, Jun, Shenzhen, 518000 (CN); Deng, Jianzhong, Shenzhen, 518000 (CN); Zhang, Xiaowen, Shenzhen, 518000 (CN); Deng, Zhiwu, Shenzhen, 518000 (CN)
(74) Representative: Grünecker Patent- und Rechtsanwälte PartG mbB

(57) **Abstract**

The present application provides a syringe positioning assembly and a syringe pump, which relate to the technical field of medical devices. The syringe positioning assembly includes a housing, a rotating member, a fixing member and a transmission member. The rotating member is connected to the fixing member via the transmission member, and during rotation, the rotating member thus can drive the fixing member to move by means of the transmission member. Specifically, when the rotating member rotates in a direction away from a carrying face of the housing, the fixing member can be moved in a direction away from a fixing face of the housing, so that while a space between the rotating member and the carrying face of the housing can facilitate the placement of a barrel of a syringe, a space between the fixing member and the fixing face of the housing can also facilitate the placement of a flange of the barrel of the syringe. Therefore, a user can move the fixing member just by operating the rotating member, so that the barrel of the syringe and the flange of the barrel of the syringe can be easily mounted and placed in corresponding positions.

## Description

### TECHNICAL FIELD

The present application relates to a syringe positioning assembly and a syringe pump, belonging to the technical field of medical devices.

### BACKGROUND ART

A positioning assembly in a syringe pump functions to fix a syringe on the syringe pump. Specifically, when fixing the syringe pump, a barrel of the syringe and a flange at an end portion of the barrel may be fixed at the same time such that the syringe can remain relatively more stable.

In the related art, corresponding components of the positioning assembly for fixing the barrel and the flange at the end portion of the barrel need to be operated and controlled separately, making the syringe fixing process complicated and inconvenient.

### SUMMARY

The present application provides a syringe positioning assembly and a syringe pump, which solve the problem in the related art of complicated and inconvenient operation process of fixing a syringe to the syringe pump.

In a first aspect, the present application provides a syringe positioning assembly, including:
a housing having a carrying face and a fixing face;
a rotating member rotatably arranged on the housing, the rotating member being configured to be rotatable in a direction toward or away from the carrying face;
a fixing member movably connected to the housing, the fixing member being opposite to the fixing face; and
a transmission member connected to the rotating member and the fixing member;
wherein when the rotating member rotates away from the carrying face, the rotating member drives the fixing member to move away from the fixing face by means of the transmission member.

In the second aspect, an embodiment of the present application further proposes another syringe positioning assembly, including:
a housing having a carrying face;
a rotating member rotatably arranged on the housing, the rotating member being configured to be rotatable in a direction toward or away from the carrying face; and
a second elastic member arranged in the housing, wherein the second elastic member is connected to both the housing and the rotating member, the second elastic member is deformed by force when the rotating member rotates away from the carrying face, and a deformation restoring force of the second elastic member is capable of driving the rotating member to rotate toward the carrying face.

In a third aspect, based on the above syringe positioning assembly, the present application also provides a syringe pump, including the above syringe positioning assembly.

In the syringe positioning assembly provided by the present application, the rotating member is connected to the fixing member via the transmission member, and during rotation, the rotating member thus can drive the fixing member to move by means of the transmission member. Specifically, when the rotating member rotates in a direction away from a carrying face of the housing, the fixing member can be moved in a direction away from a fixing face of the housing, so that while a space between the rotating member and the carrying face of the housing can facilitate the placement of a barrel of a syringe, a space between the fixing member and the fixing face of the housing can also facilitate the placement of a flange of the barrel of the syringe. Therefore, a user can move the fixing member just by operating the rotating member, so that the barrel of the syringe and the flange of the barrel of the syringe can be easily mounted and placed in corresponding positions, and finally the efficiency of fixing the syringe by the syringe positioning assembly of the present application is improved.

### BRIEF DESCRIPTION OFTHE DRAWINGS

The above and other objectives, features and advantages of the embodiments of the present application will become more readily understood by the following detailed description with reference to the accompanying drawings. Various embodiments of the present application will be illustrated by way of example and non-limitingly in the accompanying drawings, in which:
FIG. 1 is a schematic diagram of the overall structure of a syringe positioning assembly according to an embodiment of the present application;
FIG. 2 is a schematic structural diagram of a transmission member of the syringe positioning assembly according to the embodiment of the present application;
FIG. 3 is a schematic structural diagram of the assembly of the rotating member and a first transmission portion of the syringe positioning assembly according to the embodiment of the present application;
FIG. 4 is a schematic structural diagram of a fixing member of the syringe positioning assembly according to the embodiment of the present application;
FIG. 5 is a schematic diagram of the mating between a first elastic member and the fixing member of the syringe positioning assembly according to the embodiment of the present application;
FIG. 6 is a schematic diagram of the mating between a second elastic member and the rotating member of the syringe positioning assembly according to the embodiment of the present application;
FIG. 7 is a schematic structural diagram of a limiting block of the syringe positioning assembly according to the embodiment of the present application;
FIG. 8 is a schematic diagram of the mating between an angle sensor and the rotating member of the syringe positioning assembly according to the embodiment of the present application;
FIG. 9 is a schematic diagram of the second elastic member of the syringe positioning assembly according to the embodiment of the present application;
FIG. 10 is a schematic diagram of another syringe positioning assembly proposed by an embodiment of the present application;
FIG. 11 is a schematic structural diagram of a syringe positioning assembly provided by an embodiment of the present application;
FIG. 12 is a schematic structural diagram of the syringe positioning assembly shown in FIG. 11 from another perspective;
FIG. 13 is a partial schematic structural diagram of the syringe positioning assembly shown in FIG. 12;
FIG. 14 is a partial schematic structural diagram of the syringe positioning assembly shown in FIG. 12;
FIG. 15 is a schematic cross-sectional structural diagram of the syringe positioning assembly shown in FIG. 14 along A-A;
FIG. 16 is a schematic diagram of a working process of a syringe positioning assembly provided by an embodiment of the present application;
FIG. 17 is a schematic diagram of another working process of a syringe positioning assembly provided by an embodiment of the present application;
FIG. 18 is a schematic structural diagram of another syringe positioning assembly provided by the present application;
FIG. 19 is a partial schematic structural diagram of the syringe positioning assembly shown in FIG. 18;
FIG. 20 is a schematic diagram of a working process of the syringe positioning assembly shown in FIG. 19; and
FIG. 21 is a schematic diagram of another working process of the syringe positioning assembly shown in FIG. 19.

List of reference signs:
100 - Housing, 110 - Carrying face, 120 - Fixing face, 130 - Inner cavity, 140 - Blocking assembly, 141 - Blocking member, 142 - Sealing member, 150 - Second limiting structure, 160 - Stopping member,
200 - Rotating member, 210 - Handle, 211 - Connecting portion, 212 - Clamping portion, 213 - Pressing portion, 220 - First connecting shaft, 221 - Positioning member, 230 - Shifting piece, 240 - Coupling,
300 - Fixing member, 310 - Fixing piece, 320 - Second connecting shaft, 330 - Stop block, 340 - Elastic ring, 350 - Mounting block,
400 - Transmission member, 410 - First transmission portion, 420 - Second transmission portion, 421 - Guide groove, 421a - First end, 421b - Second end, 4211 - Positioning groove, 422 - Outer cylinder, 423 - Abutment piece, 424 - Push member, 424a - Abutment portion, 425 - First limiting structure, 430 - Third transmission portion, 440 - Fourth transmission portion, 441 - Positioning groove, 441a - Third end, 441b - Fourth end,
500 - Limiting block, 510 - Limiting mounting portion, 520 - Limiting portion,
600 - First elastic member,
700 - Second elastic member, 710 - Compression spring,
800 - Trigger switch,
900 - Angle sensor.

### DETAILED DESCRIPTION OF EMBODIMENTS

Embodiments of the present application will be described in detail below. Examples of the embodiments are shown in the accompanying drawings, and throughout the drawings, the same or similar reference signs refer to the same or similar elements or elements having the same or similar functions. The embodiments described below with reference to the accompanying drawings are exemplary and are intended to be illustrative of the present application, but should not be construed as limiting the present application.

A positioning assembly in a syringe pump functions to fix a syringe on the syringe pump. Specifically, when fixing the syringe pump, a barrel of the syringe and a flange at an end portion of the barrel may be fixed at the same time such that the syringe can remain relatively more stable.

In the related art, the structures of the positioning assembly for fixing the barrel and the flange at the end portion of the barrel are independent components of the positioning assembly. Therefore, when fixing the barrel and the flange at the end portion of the barrel, at least two independent components need to be operated separately. In this way, it is necessary to operate corresponding independent components to fix the barrel and the flange at the end portion of the barrel by means of the positioning assembly, making the syringe fixing process complicated and inconvenient.

In the syringe positioning assembly and the syringe pump proposed in the embodiments of the present application, a rotating member is matingly connected to a fixing member via a transmission member, so that after the rotating member is fixed to the barrel through rotation, the fixing member can move along with the rotation of the rotating member until the flange at the end portion of the barrel is fixed. In this way, the rotating member and the fixing member can both move to fix the syringe just by operating the rotating member, thereby improving the fixing efficiency of the syringe positioning assembly of the present application.

The syringe positioning assembly and the syringe pump provided by the present application will be described in detail below in conjunction with the specific embodiments.

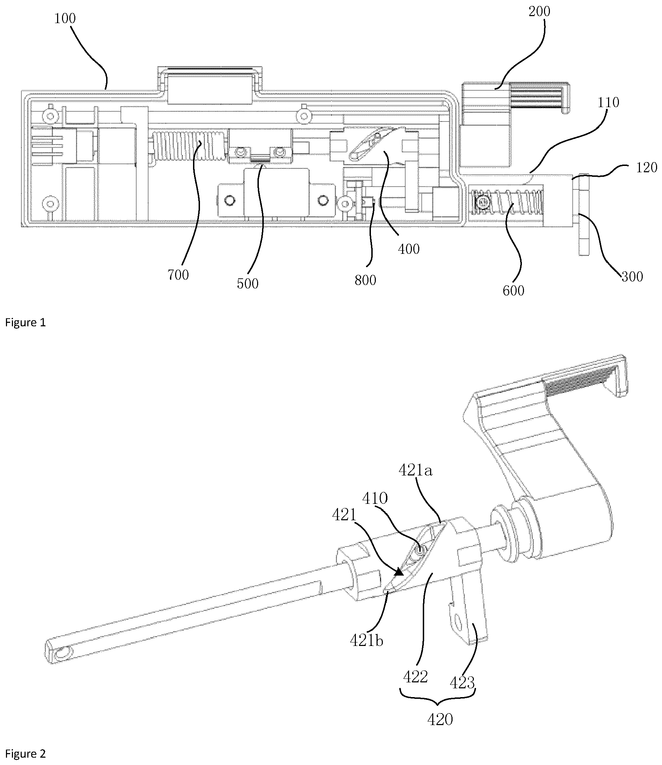

Referring to FIG. 1, an embodiment of the present application proposes a syringe positioning assembly, including a housing 100, a rotating member 200, a fixing member 300 and a transmission member 400. The syringe positioning assembly may be applied to a syringe pump.

The housing 100 is a basic member of the syringe positioning assembly of the present application. The housing 100 can provide a mounting base for at least some other components of the syringe positioning assembly. The housing 100 can also function to protect at least some other components of the syringe positioning assembly. The housing 100 may be made of metal or a composite material. The housing 100 made of metal has a better structural strength, whereas the housing 100 made of a composite material is relatively light. The specific material of the housing 100 is not limited in the present application.

The rotating member 200 and the fixing member 300 are both arranged on the housing 100 through movable connection. The rotating member 200 is rotatably connected to the housing 100. Specifically, the housing 100 may be provided with a pin hole that mates with the rotating member 200. At least part of the rotating member 200 may be arranged in the pin hole of the housing 100. The housing 100 has a carrying face 110. The carrying face 110 matches the barrel of the syringe such that the barrel of the syringe can be placed on the carrying face 110 of the housing 100. Specifically, the rotating member 200 may rotate in a direction toward the carrying face 110 of the housing 100, or in a direction away from the carrying face 110 of the housing 100. After the rotating member 200 moves in the direction away from the carrying face 110 of the housing 100, a distance between the rotating member 200 and the carrying face 110 can increase, such that a space between the rotating member 200 and the carrying face 110 is enough to easily place the barrel of the syringe on the carrying face 110; whereas after the rotating member 200 rotates toward the carrying face 110, the rotating member 200 can be rotated to press against the barrel of the syringe, so that the barrel of the syringe can be clamped between the rotating member 200 and the carrying face 110 of the housing 100, thereby achieving the purpose of fixing the barrel of the syringe.

The fixing member 300 is slidably connected to the housing 100. The housing 100 has a fixing face 120 opposite to the fixing member 300. The housing 100 can move relative to the housing 100 so that a distance between the fixing face 120 of the housing 100 and the fixing member 300 can be adjusted. Specifically, the fixing member 300 may move toward the fixing face 120 of the housing 100 so that the distance between the fixing member 300 and the fixing face 120 decreases, or may move away from the fixing face 120 so that the distance between the fixing member 300 and the fixing face 120 increases. Therefore, by increasing the distance between the fixing member 300 and the fixing face 120 of the housing 100 to be greater than the thickness of the flange at the end portion of the barrel of the syringe, the flange at the end portion of the barrel of the syringe can be easily placed between the fixing member 300 and the fixing face 120 of the housing 100. By reducing the distance between the fixing member 300 and the fixing face 120 of the housing 100, the flange of the barrel of the syringe can be clamped between the fixing member 300 and the fixing face 120, thereby achieving the purpose of fixing the flange of the barrel of the syringe.

The rotating member 200 is connected to the fixing member 300 via the transmission member 400, and during rotation, the rotating member 200 thus can drive the fixing member 300 to move by means of the transmission member 400. Specifically, when the rotating member 200 rotates in the direction away from the carrying face 110 of the housing 100, the fixing member 300 can be moved in the direction away from the fixing face 120 of the housing 100, so that while the space between the rotating member 200 and the carrying face 110 of the housing 100 can facilitate the placement of the barrel of the syringe, the space between the fixing member 300 and the fixing face 120 of the housing 100 can also facilitate the placement of the flange of the barrel of the syringe. Therefore, a user can move the fixing member 300 just by operating the rotating member 200, so that the barrel of the syringe and the flange of the barrel of the syringe can be easily mounted and placed in corresponding positions, and finally the efficiency of fixing the syringe by the syringe positioning assembly of the present application is improved.

In addition, it should be understood that in some implementations, the fixing member 300 may be configured to drive the rotating member 200 to rotate during the movement. Specifically, when the user operates the fixing member 300 to move in the direction away from the fixing face 120 of the housing 100, the fixing member 300 can drive the rotating member 200 to rotate in the direction away from the carrying face 110 of the housing 100 by means of the transmission member 400. In this way, the user can make the rotating member 200 rotate accordingly just by operating the fixing member 300, so that the barrel of the syringe and the flange of the barrel of the syringe can be easily mounted and placed in corresponding positions, and finally the efficiency of fixing the syringe by the syringe positioning assembly of the present application is improved.

In some implementations, still referring to FIG. 1, in order to make the structure of the syringe positioning assembly of the present application more compact, the housing 100 of the present application may be provided as a structural member having an inner cavity, and the transmission member 400 may be arranged in the housing 100. In this way, the transmission member 400 can utilize the space inside the housing 100, and the housing 100 can also function to protect the transmission member 400, so as to prevent the transmission member 400 from being exposed and damaged, or to prevent impurities from entering the transmission member 400 to damage the transmission member 400, so that the rotating member 200 is drivingly and matingly connected to the fixing member 300 via the transmission member 400 in a more stable and reliable manner. Correspondingly, the rotating member 200 may pass through the housing 100 and be connected to the transmission member 400, and part of the rotating member 200 is located outside the housing 100 to facilitate the user to operate the rotating member 200 to rotate. The fixing member 300 may alternatively pass through the housing 100 and the transmission member 400, and part of the fixing member 300 is located outside the housing 100, so that the user can easily observe the clamping effect of the rotating member 200 and the fixing member 300 on the syringe.

In some implementations, referring to FIG. 2, in order to enable the transmission member 400 of the present application to convert a rotational acting force of the rotating member 200 into an acting force that drives the fixing member 300 to move, the transmission member 400 may specifically include a first transmission portion 410 and a second transmission portion 420. The first transmission portion 410 is connected to and mates with the second transmission portion 420, the first transmission portion 410 is connected to the rotating member 200, and the second transmission portion 420 is connected to the fixing member 300. Therefore, the first transmission portion 410 can rotate with the rotating member 200. After rotating along with the rotating member 200, the first transmission portion 410 can drive the second transmission portion 420 to move, such that the fixing member 300 can move in the direction away from the fixing face 120 of the housing 100. Specifically, the direction in which the fixing member 300 moves away from the fixing face 120 of the housing 100 is defined as a first direction, the direction in which the fixing member 300 moves toward the fixing face 120 of the housing 100 is defined as a second direction, the direction in which the rotating member 200 rotates away from the carrying face 110 of the housing 100 is defined as a third direction, and the direction in which the rotating member 200 rotates toward the carrying face 110 of the housing 100 is defined as a fourth direction. When the first transmission portion 410 rotates, the first transmission portion 410 can drive the second transmission portion 420 to move in the first direction, such that the second transmission portion 420 can drive the fixing member 300 to also move in the first direction, that is, driving the fixing member 300 to move in the direction away from the fixing face 120 of the housing 100.

In some implementations, still referring to FIG. 2, in order to enable the first transmission portion 410 to matingly connected to the second transmission portion 420 to convert the rotational force into an acting force of linear motion, the second transmission portion 420 may be provided with a guide groove 421. The groove profile of the guide groove 421 mates with the first transmission portion 410, such that at least part of the first transmission portion 410 can be embedded in the guide groove 421. The guide groove 421 has a first end 421a and a second end 421b. After the first transmission portion 410 rotates in the third direction, the first transmission portion 410 may also move relative to the second transmission portion 420 in a direction from the first end 421a to the second end 421b of the guide groove 421, so that the fixing member 300 can be driven to move in the first direction. When the first transmission portion 410 is embedded in the guide groove 421 of the second transmission portion 420, the guide groove 421 can function to limit the first transmission portion 410, so that when the first transmission portion 410 rotates in the first direction, the first transmission portion 410 can always be located in the guide groove 421, and the mating relationship between the first transmission portion 410 and the second transmission portion 420 thus can be relatively stable.

Of course, in other implementations, in order to achieve the mating between the first transmission portion 410 and the second transmission portion 420 for transmission, it is also possible to provide the guide groove 421 in the first transmission portion 410. Correspondingly, at least part of the second transmission portion 420 may be embedded in the guide groove 421 of the first transmission portion 410. In this way, after the first transmission portion 410 rotates in the third direction, the second transmission portion 420 may move relative to the first transmission portion 410 in the direction from the first end 421a to the second end 421b of the guide groove 421, so that the fixing member 300 can be driven to move in the first direction.

In some implementations, referring to FIG. 2, the third direction of rotation of the rotating member 200 in the present application may be arranged around the first direction of movement of the fixing member 300, that is, the first direction is the axis of a rotation trajectory of the rotating member 200, and the guide groove 421 may be arranged to be arranged spirally around the first direction on the second transmission portion 420, so that the guide groove 421 can not only be distributed in the first direction, but the guide groove 421 can also be distributed in the third direction. Therefore, during the movement of the first transmission portion 410 relative to the second transmission portion 420 along the guide groove 421, the rotational force of the first transmission portion 410 can act on an inner wall of the guide groove 421, such that the rotational force of the first transmission portion 410 can be decomposed into a linear driving force acting on the second transmission portion 420 in the first direction, the second transmission portion 420 thus moves relative to the first transmission portion 410 in the first direction, and finally the fixing member 300 moves in the direction away from the fixing face 120 of the housing 100.

In addition, in other implementations, it is also possible that the second transmission portion 420 is provided with a guide rail protruding structure distributed spirally around the first direction. Correspondingly, the first transmission portion 410 may be provided with a groove structure that mates with the guide rail protruding structure, such that the first transmission portion 410 and the second transmission portion 420 can be embedded in each other, and the rotation of the first transmission portion 410 can also drive the second transmission portion 420 to move in the first direction.

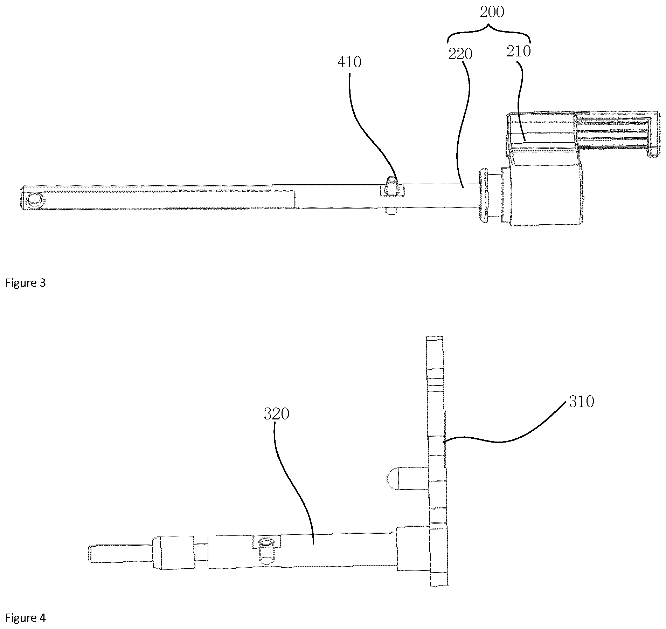

In some implementations, referring to FIG. 3, the rotating member 200 of the present application specifically includes a handle 210 and a first connecting shaft 220. The handle 210 is located outside the housing 100. One end of the first connecting shaft 220 is connected to the handle 210, and the other end of the first connecting shaft 220 passes through the housing 100 and extends into the housing 100. The first transmission portion 410 is connected to the part of the first connecting shaft 220 that is located inside the housing 100. The user can hold the handle 210 outside the housing 100 to cause the first connecting shaft 220 to rotate, so as to drive the first transmission portion 410 to rotate. The carrying face 110 of the housing 100 is located at an outside surface of the housing 100. When the rotating member 200 rotates in the third direction, the handle 210 can rotate away from the carrying face 110 of the housing 100. When the rotating member 200 rotates in the fourth direction, the handle 210 can toward the carrying face 110 of the housing 100 such that the barrel. Therefore, the barrel of the syringe can be mounted between the handle 210 and the carrying face 110 of the housing 100, and the barrel of the syringe can be clamped between the handle 210 and the carrying face 110 of the housing 100.

Providing the handle 210 outside the housing 100 can not only serve the purpose of fixing the barrel of the syringe, but can also enable the user to easily operate the rotating member 200 to rotate, thereby making the syringe positioning assembly of the present application more convenient to use and operate.

Referring to FIG. 3, the first transmission portion 410 of the present application may be specifically configured as a pin fixed to a side wall of the first connecting shaft 220. Specifically, the side wall of the first connecting shaft 220 may be provided with a pin hole that mates with the pin, such that the pin can be inserted into the pin hole of the first connecting shaft 220. In this way, when the pin is mounted on the first connecting shaft 220, the pin can be configured to protrude relative to the side wall of the first connecting shaft 220. The pin may be removably connected to the first connecting shaft 220. Specifically, the pin may be in transition fit with the pin hole of the first connecting shaft 220, such that the pin can be fixed to the first connecting shaft 220, and the pin can be relatively easily removed from the first connecting shaft 220. The removable connection between the pin and the first connecting shaft 220 can reduce the difficulty of preparing the first connecting shaft 220 and make the rotating member 200 and the transmission member 400 easy to disassemble and maintain. In addition, the first transmission portion 410 may have an integral structure with the rotating member 200.

Referring to FIG. 2, the second transmission portion 420 may specifically adopt a sleeve structure, the second transmission portion 420 is sleeved on the first connecting shaft 220, and the second transmission portion 420 can rotate relative to the first connecting shaft 220. Where the second transmission portion 420 is sleeved on the first connecting shaft 220, the pin can be embedded in the guide groove 421 of the second transmission portion 420, so that the first transmission portion 410 and the second transmission portion 420 can be arranged relatively close to each other. This makes the structure of the transmission member 400 more compact, and finally the structure of the syringe positioning assembly of the present application can also be relatively more compact. The guide groove 421 of the second transmission portion 420 may be configured to pass through a side wall of the second transmission portion 420, so that the user can easily observe whether the pin and the guide groove 421 of the second transmission portion 420 are mated in place, so that in the event of a fault of the transmission member 400, the user can easily troubleshoot the fault.

In the present application, the length of the pin may be set to be larger than the outer diameter of the first connecting shaft 220. Therefore, when the pin passes through the first connecting shaft 220, two ends of the pin can each protrude from a side wall of the first connecting shaft 220. Correspondingly, there may be two guide grooves 421 of the second transmission portion 420. The two guide grooves 421 may respectively mate with the two ends of the pin, so that the two ends of the pin can be respectively embedded in the two guide grooves 421. In this way, the mating connection relationship between the first transmission portion 410 and the second transmission portion 420 can be more balanced and stable.

In addition, it should be understood that in other implementations, where the second transmission portion 420 is sleeved on the first connecting shaft 220, if the second transmission portion 420 is provided with a guide rail protruding structure distributed spirally around the first direction, the guide rail protruding structure may be arranged on an inner wall of the second transmission portion 420.

In some implementations, referring to FIGS. 1 and 4, the fixing member 300 of the present application may specifically include a fixing piece 310 and a second connecting shaft 320. The fixing piece 310 is located outside the housing 100, and the fixing piece 310 is opposite to the fixing face 120 of the housing 100. One end of the second connecting shaft 320 is connected to the fixing piece 310, and the other end of the second connecting shaft 320 passes through the housing 100 and is connected to the second transmission portion 420 in the housing 100. When the second transmission portion 420 moves in the first direction, the second connecting shaft 320 can move in the first direction along with the second transmission portion 420, so that the fixing piece 310 connected to the second connecting shaft 320 can also move in the first direction. In this way, the fixing piece 310 moves in the direction away from the fixing face 120 of the housing 100, so that there is enough space between the fixing piece 310 and the fixing face 120 for the flange of the barrel of the syringe to be placed.

In some implementations, referring to FIG. 2, in order to enable the second transmission portion 420 to be connected to the second connecting shaft 320 in the present application, the second transmission portion 420 may specifically include an outer cylinder 422 and an abutment piece 423. The outer cylinder 422 is sleeved on the first connecting shaft 220. Correspondingly, the guide groove 421 is also provided on the outer cylinder 422. The abutment piece 423 is connected to an outer wall of the outer cylinder 422, and the abutment piece 423 is sleeved on the second connecting shaft 320. The second connecting shaft 320 is provided with a boss. The boss of the second connecting shaft 320 is opposite to the abutment piece 423. Therefore, when the abutment piece 423 moves in the first direction, the abutment piece 423 may abut against the boss. In this way, the abutment piece 423 can push the second connecting shaft 320 to move in the first direction, and further push the fixing piece 310 to move in a direction away from a mounting face of the housing 100.

Specifically, the abutment piece 423 is movably sleeved on the second connecting shaft 320, and the abutment piece 423 thus can move relative to the second connecting shaft 320. In this way, the abutment piece 423 can be moved in a direction away from the boss, such that the abutment piece 423 can be removed from the second connecting shaft 320, so that the abutment piece 423 and the second connecting shaft 320 can be easily disassembled and assembled. Of course, in other implementations, in order to ensure reliable connection between the second transmission portion 420 and the second connecting shaft 320, the abutment piece 423 may alternatively be fixedly connected to the second connecting shaft 320.

In some implementations, referring to FIGS. 1 and 2, in order to make the operation and use of the syringe positioning assembly of the present application more convenient, it is also possible that when the rotating member 200 rotates toward the carrying face 110 of the housing 100, the fixing member 300 can move simultaneously toward the fixing face 120 of the housing 100. In this way, when the rotating member 200 rotates toward the carrying face 110 of the housing 100 to fix the barrel of the syringe, the fixing member 300 can also move toward the fixing face 120 of the housing 100, so that the fixing member 300 can fix the barrel of the syringe. Therefore, while the syringe positioning assembly of the present application can fix the barrel of the syringe, the flange of the barrel of the syringe can also be fixed simultaneously, making the use and operation of the syringe positioning assembly more convenient.

Specifically, when the fixing member 300 moves in the second direction, the second transmission portion 420 can also move in the second direction. During this process, the first transmission portion 410 moves relative to the second transmission portion 420 in a direction from the second end 421b to the first end 421a of the guide groove 421, and the first transmission portion 410 can rotate in the fourth direction, so that the rotating member 200 can rotate in the fourth direction, so that the user can drive the fixing member 300 to move in the second direction to enable the rotating member 200 to rotate in the fourth direction synchronously. Of course, in other implementations, it is also possible to apply a rotational force to the rotating member 200 in the fourth direction such that the first transmission portion 410 can also rotate in the fourth direction. During this process, the first transmission portion 410 moves relative to the second transmission portion 420 in the direction from the second end 421b to the first end 421a of the guide groove 421, and the second transmission portion 420 can correspondingly move in the second direction, so that the fixing member 300 can also move in the second direction. In this way, the purpose of synchronously driving the rotating member 200 and the fixing member 300 to restore can be achieved.

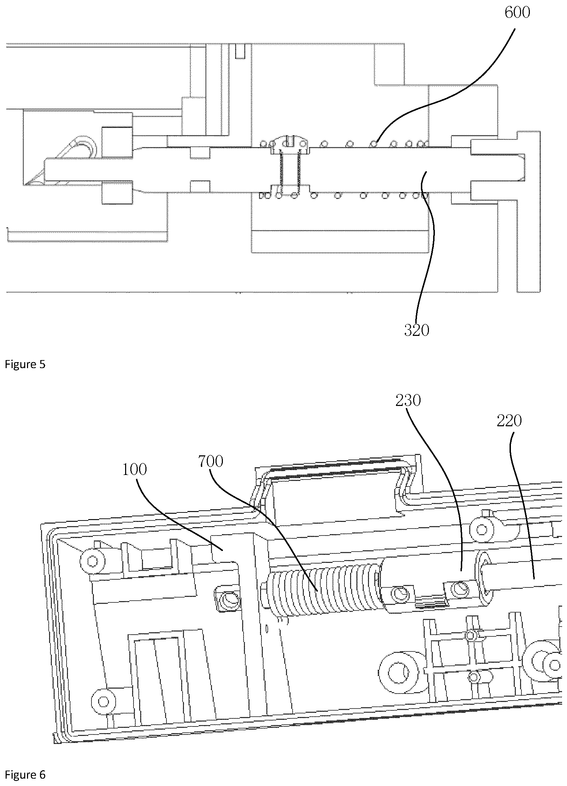

Specifically, referring to FIGS. 1 and 5, the syringe positioning assembly of the present application may be further configured to include a first elastic member 600. The first elastic member 600 may be connected to the fixing member 300 and the housing 100. When the fixing member 300 moves away from the fixing face 120 of the housing 100, that is, when the fixing member 300 moves in the first direction, the first elastic member 600 can be deformed by the acting force of the fixing member 300. In this way, when the fixing member 300 is no longer subjected to the acting force in the first direction, a deformation restoring force of the first elastic member 600 can drive the fixing member 300 to move toward the fixing face 120 of the housing 100, that is, the fixing member 300 moves in the second direction, so that the fixing member 300 can clamp the flange of the barrel of the syringe. Therefore, when the distance between the fixing member 300 and the fixing face 120 of the housing 100 increases such that the flange of the barrel of the syringe can be placed between the fixing member 300 and the fixing face 120 of the housing 100, the user only needs to cancel the acting force applied to the fixing member 300 in the first direction in order to enable the fixing member 300 to automatically move in the second direction to clamp the flange of the barrel of the syringe, thereby greatly simplifying the operation of the syringe positioning assembly of the present application.

Referring to FIG. 5, the first elastic member 600 may specifically take the form of a spring. The first elastic member 600 may be sleeved on the second connecting shaft 320. A side wall of the second connecting shaft 320 is provided with a bump that can be embedded in a gap of the first elastic member 600. In this way, the first elastic member 600 can be fixed to the second connecting shaft 320, and one end of the first elastic member 600 can abut against an inner wall of the housing 100. Therefore, when the fixing member 300 is forced to move in the first direction, the first elastic member 600 may be compressed, and the deformation restoring of the first elastic member 600 may push the fixing member 300 to move in the second direction. Of course, in other implementations, where the first elastic member 600 is fixedly sleeved on the second connecting shaft 320, when the fixing member 300 is forced to move in the first direction, the first elastic member 600 may alternatively be stretched, and the deformation restoring of the first elastic member 600 may pull the fixing member 300 to move in the second direction.

In addition, the first elastic member 600 being sleeved on the second connecting shaft 320 can also make the connection structure between the first elastic member 600 and the second connecting shaft 320 relatively more compact and occupy less space, so that the structure of the syringe positioning assembly of the present application can also be relatively more compact.

In some implementations, referring to FIGS. 1, 5 and 6, in order to further facilitate the operation of the syringe positioning assembly of the present application, the syringe positioning assembly may be further configured to include a second elastic member 700. The second elastic member 700 may be connected to the rotating member 200 and the housing 100. When the rotating member 200 rotates away from the carrying face 110 of the housing 100, that is, when the rotating member 200 rotates in the third direction, the second elastic member 700 can be deformed by the acting force of the rotating member 200. In this way, when the rotating member 200 is no longer subjected to the acting force in the third direction, a deformation restoring force of the second elastic member 700 can drive the rotating member 200 to move toward the carrying face 110 of the housing 100, that is, the rotating member 200 rotates in the fourth direction, so that the rotating member 200 can clamp the barrel of the syringe. Therefore, when the distance between the rotating member 200 and the carrying face 110 of the housing 100 increases such that the barrel of the syringe can be placed between the rotating member 200 and the carrying face 110 of the housing 100, the user only needs to cancel the acting force applied to the rotating member 200 in the third direction in order to enable the rotating member 200 to automatically rotate in the fourth direction to clamp the barrel of the syringe, thereby greatly simplifying the operation of the syringe positioning assembly of the present application.

Specifically, the second elastic member 700 may take the form of a torsion spring. The second elastic member 700 may be sleeved on the first connecting shaft 220. One end of the second elastic member 700 is fixed to the inner wall of the housing 100, and the other end of the second elastic member 700 is fixed to the first connecting shaft 220. When the user applies a rotational force to the rotating member 200 in the third direction to rotate the first connecting shaft 220, the second elastic member 700 can rotate along therewith and be twisted and compressed. After the user no longer applies the acting force to the rotating member 200 in the third direction, the deformation restoring force of the second elastic member 700 can drive the first connecting shaft 220 to rotate in the fourth direction, such that the rotating member 200 rotates toward the carrying face 110 of the housing 100 to fix the barrel of the syringe.

In addition, the second elastic member 700 being sleeved on the first connecting shaft 220 can also make the connection structure between the second elastic member 700 and the first connecting shaft 220 relatively more compact and occupy less space, so that the structure of the syringe positioning assembly of the present application can also be relatively more compact.

Therefore, during the actual use of the syringe positioning assembly of the present application, after the user applies an acting force to the rotating member 200 in the third direction to rotate the rotating member 200 away from the carrying face 110 of the housing 100, the fixing member 300 can synchronously move away from the fixing face 120 of the housing 100, and then the barrel of the syringe can be mounted and placed between the rotating member 200 and the carrying face 110 of the housing 100. Correspondingly, the flange of the barrel can be mounted and placed between the fixing member 300 and the fixing face 120 of the housing 100. During this process, the acting force to the rotating member 200 in the third direction applied by the user can act on the first elastic member 600 and the second elastic member 700, such that the first elastic member 600 and the second elastic member 700 are each in a compressed and deformed state. After the user cancels the acting force applied to the rotating member 200 in the third direction, the deformation restoring force of the first elastic member 600 can drive the fixing member 300 to move in the second direction, and the deformation restoring force of the second elastic member 700 can drive the rotating member 200 to rotate in the fourth direction, so that the rotating member 200 can automatically press against the barrel of the syringe, and the fixing member 300 can automatically press against the flange of the barrel of the syringe.

In addition, it should be understood that when the deformation restoring force of the first elastic member 600 drives the fixing member 300 to move in the second direction, the fixing member 300 can also push the second transmission portion 420 to move in the second direction. In this way, the second transmission portion 420 can also drive the first transmission portion 410 to rotate in the fourth direction, so that the rotating member 200 connected to the first transmission portion 410 can rotate in the fourth direction. When the deformation restoring force of the second elastic member 700 drives the rotating member 200 to rotate in the fourth direction, the rotating member 200 can also drive the first transmission portion 410 to rotate in the fourth direction. In this way, the first transmission portion 410 can also drive the second transmission portion 420 to rotate in the second direction, so that the fixing member 300 connected to the second transmission portion 420 can move in the second direction. Therefore, the deformation restoring effects of the first elastic member 600 and the second elastic member 700 can also be superimposed on each other, so that the restoring effects on the rotating member 200 and the fixing member 300 are better.

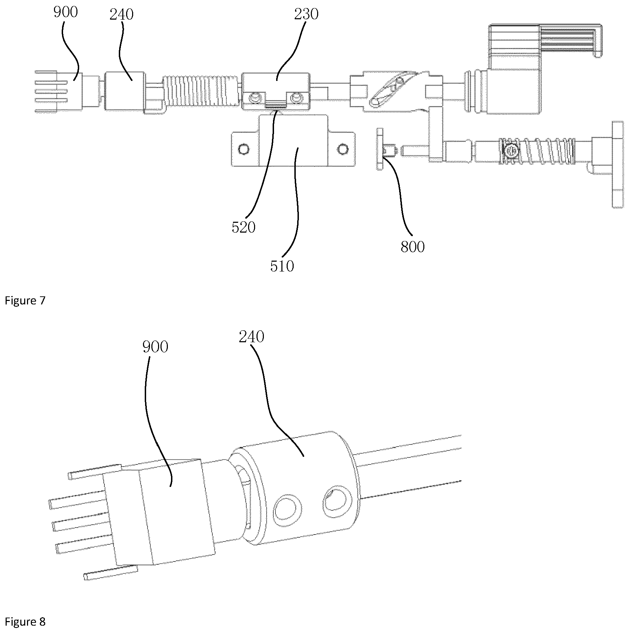

In some implementations, in order to prevent the rotating member 200 from rotating excessively and the fixing member 300 from moving excessively, referring to FIGS. 1 and 7, the syringe positioning assembly of the present application may be further provided with a limiting block 500. The limiting block 500 may be fixed to the inner wall of the housing 100, and the rotating member 200 can be further provided with a shifting piece 230. The shifting piece 230 is connected to the first connecting shaft 220, so when the first connecting shaft 220 rotates, the shifting piece 230 can rotate along therewith. The limiting block 500 may be specifically located on a rotation trajectory of the shifting piece 230. Therefore, the limiting block 500 can limit the shifting piece 230 in a rotation direction of the shifting piece 230, so as to determine the maximum angle by which the rotating member 200 can rotate, and the maximum distance by which the fixing member 300 can move relative to the fixing face 120 of the housing 100. Specifically, in the present application, when the rotating member 200 rotates 120 degrees, the shifting piece 230 can abut against the limiting block 500 to prevent the rotating member 200 from continuing to rotate.

In addition, referring to FIGS. 7 and 8, the syringe positioning assembly of the present application may be further provided with an angle sensor 900. The angle sensor 900 may be arranged in the housing 100, and the angle sensor 900 may be connected to the first connecting shaft 220. In this way, the user can also obtain a rotation angle of the first connecting shaft 220, then obtain a movement stroke of the fixing member 300, and finally obtain a distance between the fixing piece 310 and the fixing face 120 of the housing 100, to allow the user to determine whether the flange of the barrel of the syringe can be placed between the fixing piece 310 and the fixing face 120 of the housing 100. The angle sensor 900 may be specifically arranged on the inner wall of the housing 100, and the first connecting shaft 220 may be specifically connected to the angle sensor 900 via a coupling 240. The angle sensor 900 may specifically be a rotary potentiometer.

Specifically, in the present application, the maximum rotation angle of the rotating member 200 is 120 degrees. That is, when the rotating member 200 rotates 120 degrees, the limiting block 500 and the shifting piece 230 limit each other.

In some implementations, in order to detect whether the syringe is mounted on the syringe positioning assembly of the present application, referring to FIGS. 1 and 7, the syringe positioning assembly may be further provided with a trigger switch 800. The trigger switch 800 may be arranged in the housing 100 and opposite to the fixing member 300. Therefore, the fixing member 300 can be in contact with or separated from the trigger switch 800 during the movement. When the fixing member 300 is in contact with the trigger switch 800, the trigger switch 800 is triggered to be turned on and has a corresponding signal. When the fixing member 300 is separated from the trigger switch 800, the trigger switch 800 is turned off, and the corresponding signal disappears.

In the present application, the trigger switch 800 may be specifically opposite to an end portion of the second connecting shaft 320 away from the fixing piece 310. When the distance between the fixing piece 310 of the fixing member 300 and the fixing face 120 of the housing 100 is minimal, the end portion of the second connecting shaft 320 can act on the trigger switch 800, such that the trigger switch 800 is turned on and generates a corresponding signal, which means that the flange of the barrel of the syringe is placed between the fixing piece 310 and the fixing face 120 of the housing 100 at this time. When the fixing piece 310 moves in the first direction, the second connecting shaft 320 can then move along therewith in the second direction until the end portion of the second connecting shaft 320 is separated from the trigger switch 800, so that the corresponding signal of the trigger switch 800 disappears. When the flange of the barrel of the syringe is clamped between the fixing piece 310 and the housing 100, the end portion of the second connecting shaft 320 is still separated from the trigger switch 800, and the trigger switch 800 thus continues to have no signal, it can be determined that the flange of the barrel of the syringe has been mounted between the fixing piece 310 and the fixing face 120 of the housing 100, thereby facilitating the user to accurately determine the mounting state of the syringe.

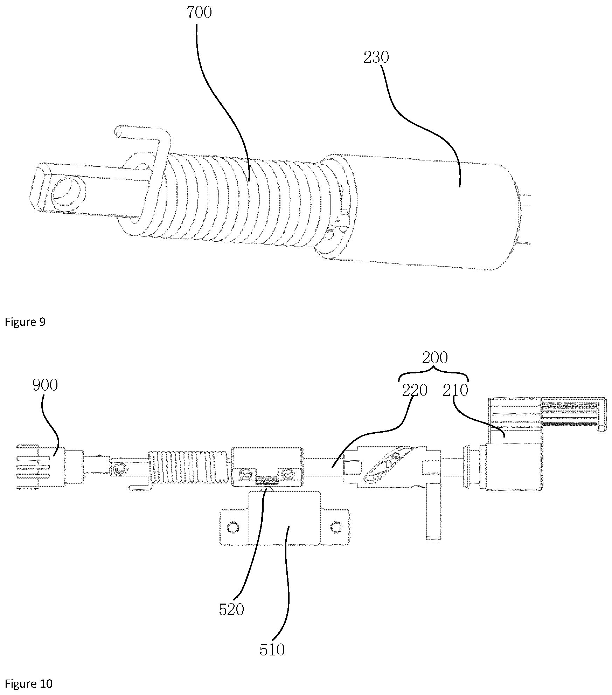

Referring to FIG. 10, the present application also proposes a syringe positioning assembly, which includes a housing 100, a rotating member 200 and a second elastic member 700. The second elastic member 700 may be connected to the rotating member 200 and the housing 100. When the rotating member 200 rotates away from the carrying face 110 of the housing 100, that is, when the rotating member 200 rotates in the third direction, the second elastic member 700 can be deformed by the acting force of the rotating member 200. In this way, when the rotating member 200 is no longer subjected to the acting force in the third direction, a deformation restoring force of the second elastic member 700 can drive the rotating member 200 to move toward the carrying face 110 of the housing 100, that is, the rotating member 200 rotates in the fourth direction, so that the rotating member 200 can clamp the barrel of the syringe. Therefore, when the distance between the rotating member 200 and the carrying face 110 of the housing 100 increases such that the barrel of the syringe can be placed between the rotating member 200 and the carrying face 110 of the housing 100, the user only needs to cancel the acting force applied to the rotating member 200 in the third direction in order to enable the rotating member 200 to automatically rotate in the fourth direction to clamp the barrel of the syringe, thereby greatly simplifying the operation of the syringe positioning assembly of the present application.

Specifically, the second elastic member 700 may take the form of a torsion spring. The second elastic member 700 may be sleeved on the rotating member 200. One end of the second elastic member 700 is fixed to the inner wall of the housing 100, and the other end of the second elastic member 700 is fixed to the rotating member 200. When the user applies a rotational force to the rotating member 200 in the third direction to rotate the rotating member 200, the second elastic member 700 can rotate along therewith and be twisted and compressed. After the user no longer applies the acting force to the rotating member 200 in the third direction, the deformation restoring force of the second elastic member 700 can drive the rotating member 200 to rotate in the fourth direction, such that the rotating member 200 rotates toward the carrying face 110 of the housing 100 to fix the barrel of the syringe.

In addition, the second elastic member 700 being sleeved on the rotating member 200 can also make the connection structure between the second elastic member 700 and the rotating member 200 relatively more compact and occupy less space, so that the structure of the syringe positioning assembly of the present application can also be relatively more compact.

In some implementations, the syringe positioning assembly of the present application may be further configured to include a limiting block 500 and an angle sensor 900. The limiting block 500 may be fixed to the inner wall of the housing 100, and the rotating member 200 can be further provided with a shifting piece 230. The shifting piece 230 is connected to the first connecting shaft 220, so when the first connecting shaft 220 rotates, the shifting piece 230 can rotate along therewith. The limiting block 500 may be specifically located on a rotation trajectory of the shifting piece 230. Therefore, the limiting block 500 can limit the shifting piece 230 in a rotation direction of the shifting piece 230, so as to determine the maximum angle by which the rotating member 200 can rotate, and the maximum distance by which the fixing member 300 can move relative to the fixing face 120 of the housing 100. Specifically, in the present application, when the rotating member 200 rotates 120 degrees, the shifting piece 230 can abut against the limiting block 500 to prevent the rotating member 200 from continuing to rotate.

In addition, referring to FIGS. 7 and 8, the syringe positioning assembly of the present application may be further provided with an angle sensor 900. The angle sensor 900 may be arranged in the housing 100, and the angle sensor 900 may be connected to the first connecting shaft 220. In this way, the user can also obtain a rotation angle of the first connecting shaft 220, then obtain a movement stroke of the fixing member 300, and finally obtain a distance between the fixing piece 310 and the fixing face 120 of the housing 100, to allow the user to determine whether the flange of the barrel of the syringe can be placed between the fixing piece 310 and the fixing face 120 of the housing 100. The angle sensor 900 may be specifically arranged on the inner wall of the housing 100, and the first connecting shaft 220 may be specifically connected to the angle sensor 900 via a coupling 240. The angle sensor 900 may specifically be a rotary potentiometer.

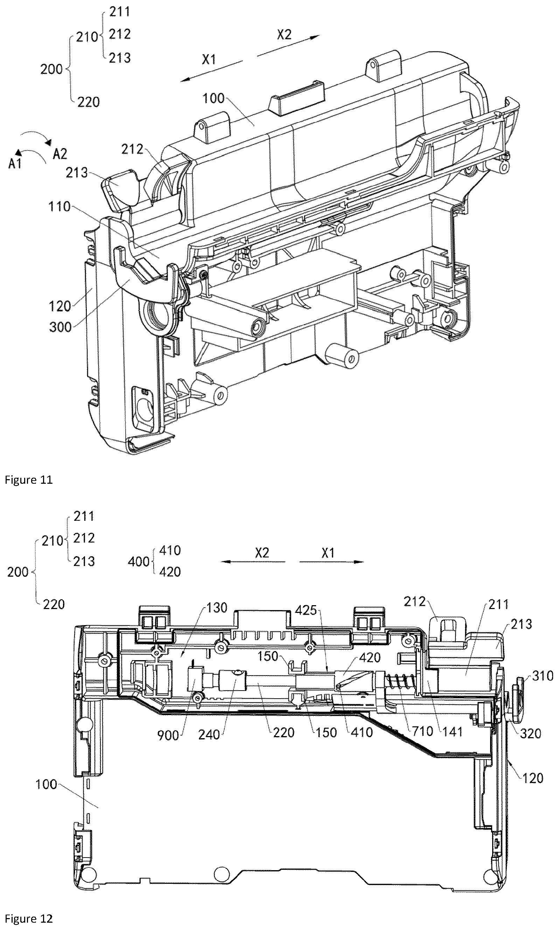

FIGS. 11-21 show schematic structural diagrams of a syringe positioning assembly provided by another embodiment of the present application. In the embodiment shown in FIGS. 11-21, the syringe positioning assembly includes a compression spring. By providing the compression spring, after the barrel of the syringe is placed between the handle and the carrying face, it is only necessary to cancel the acting force applied to the handle in order to enable the handle to be automatically moved to clamp the barrel of the syringe under the action of the deformation restoring force of the compression spring, so that the process of clamping the barrel of the syringe by the handle is effectively simplified, and the adaptive process of clamping barrels of syringes of different sizes by the syringe positioning assembly is implemented, making it convenient for the user to use.

The embodiment of the present application will be described below with reference to the figures for the embodiment of the present application.

As shown in FIGS. 11 and 12, FIG. 11 is a schematic structural diagram of a syringe positioning assembly provided by an embodiment of the present application. FIG. 12 is a schematic structural diagram of the syringe positioning assembly shown in FIG. 11 from another perspective.

The syringe pump may include a syringe positioning assembly. The syringe pump is a medical device for controlling an injection speed of a liquid drug in the syringe. The syringe positioning assembly may be configured to fix the syringe in the syringe pump to achieve stable mounting of the syringe.

The syringe positioning assembly provided by the embodiments of the present application can not only be used in syringe pumps, but can also be used in other apparatuses in other embodiments to fix structural members similar in shape to the syringe. This is not limited in the present application. In the embodiment of the present application, an example is taken for description, in which the syringe positioning assembly is configured to fix a syringe (not shown in FIGS. 11 and 12), and the syringe includes a barrel in the shape of a hollow cylinder and a flange fixed to an end portion of the barrel and protruding relative to an outer surface of the barrel. In other embodiments, the syringe may be in other shapes. This is not limited in the present application.

The syringe positioning assembly may include a housing 100. The housing 100 may be made of metal, plastic, or a composite of multiple materials. This is not limited in the present application. The housing 100 is a basic member of the syringe positioning assembly, and can provide a mounting base for at least some other components of the syringe positioning assembly, providing a strength support function.

As an example, the housing 100 may have an inner cavity 130, and some other components of the syringe positioning assembly may be received in the inner cavity 130. The housing 100 can function to protect the components located in the inner cavity 130, and can prevent external factors such as water or dust from entering the inner cavity 130, which otherwise affects the service life of the internal components of the syringe positioning assembly.

As an example, the housing 100 may have a carrying face 110. The carrying face 110 may be configured to carry the syringe. Specifically, the carrying face 110 may be configured to carry the barrel of the syringe. The shape of the carrying face 110 may be adapted to the shape of the outer surface of the barrel of the syringe. When the barrel of the syringe is fixed to the carrying face 110, the contact area between the carrying face 110 and the barrel of the syringe can then be effectively increased, facilitating the improvement of the reliability of the syringe positioning assembly in fixing the syringe. As an example, a part of the barrel of the syringe that faces the carrying face 110 may be in the form of a convex arc-shaped surface. Adaptively, the carrying face 110 may be in the form of a concave arc-shaped surface to adapt to the shape of the outer surface of the barrel of the syringe. In other embodiments, the shape of the carrying face 110 may not be adapted to the shape of the outer surface of the barrel of the syringe. This is not limited in the present application.

As an example, the housing 100 may have a fixing face 120. The fixing face 120 may be configured to fix the flange of the syringe. The shape of the fixing face 120 may match the shape of a surface of the flange of the syringe that faces the fixing face 120. When the flange of the syringe is fixed to the fixing face 120, the contact area between the fixing face 120 and the flange of the syringe can then be effectively increased, also facilitating the improvement of the reliability of the syringe positioning assembly in fixing the syringe. As an example, the surface of the flange of the syringe that faces the fixing face 120 may be in the form of a plane. Adaptively, the fixing face 120 may also in the form of a plane. In other embodiments, the shape of the fixing face 120 may not be adapted to the shape of the flange of the syringe. This is not limited in the present application.

As shown in FIGS. 11, 12 and 13, FIG. 13 is a partial schematic structural diagram of the syringe positioning assembly shown in FIG. 12.

In some embodiments, the syringe positioning assembly may further include a rotating member 200. The rotating member 200 may include a handle 210 and a first connecting shaft 220 which are connected to each other. The first connecting shaft 220 may be rotatably arranged in the housing 100, the handle 210 may be located outside the housing 100, and the handle 210 is configured to clamp the syringe to the carrying face 110. Specifically, the user may rotate the handle 210 in the direction toward the carrying face 110, or rotate the handle 210 in the direction away from the carrying face 110. When the handle 210 is rotated in the direction away from the carrying face 110 of the housing 100, the distance between the handle 210 and the carrying face 110 can increase, thereby releasing the space between the handle 210 and the carrying face 110, so that the barrel of the syringe can be smoothly mounted on the carrying face 110. When the handle 210 is rotated in the direction toward the carrying face 110, the distance between the handle 210 and the carrying face 110 can decrease. When the syringe has been placed on the carrying face 110, by rotating the handle 210 toward the carrying face 110, the handle 210 can be rotated to press against the barrel of the syringe. In this way, the barrel of the syringe can be clamped between the handle 210 and the carrying face 110, thereby achieving the fixation of the barrel of the syringe by the syringe positioning assembly. In the embodiment of the present application, by providing the handle 210 located outside the housing 100, the user can operate easily to implement the process of clamping the barrel of the syringe.

As an example, the handle 210 may include a connecting portion 211 and a clamping portion 212. The connecting portion 211 may be fixed to the first connecting shaft 220. The clamping portion 212 is configured to clamp the syringe at the carrying face 110, and the clamping portion 212 may be bent toward the carrying face 110 relative to the connecting portion 211. The shape of a surface of the clamping portion 212 that faces the carrying face 110 may be adapted to the shape of the outer surface of the barrel of the syringe. As an example, the surface of the barrel of the syringe that faces the clamping portion 212 may be a convex arc-shaped surface, and correspondingly, the surface of the clamping portion 212 that faces the carrying face 110 may be a concave arc-shaped surface, so that when the barrel of the syringe is fixed between the clamping portion 212 and the carrying face 110, the contact area between the clamping portion 212 and the barrel of the syringe can be effectively increased, facilitating the improvement of the reliability of the clamping portion 212 mating with the carrying face 110 to fix the syringe. In other embodiments, the shape of the surface of the clamping portion 212 that faces the carrying face 110 may alternatively be V-shaped, arc-shaped, or a combination of multiple shapes. This is not limited in the present application.

As an example, the handle 210 may further include a pressing portion 213. The pressing portion 213 may be connected to the connecting portion 211, is located on a side of the clamping portion 212 away from the carrying face 110, and is inclined relative to the clamping portion 212. By providing the pressing portion 213, an operating space can be provided for the user, so that when a space between the clamping portion 212 and the syringe is small, for example, when the clamping portion 212 clamps the syringe, the user can rotate the handle 210 by actuating the pressing portion 213, so as to release the syringe.

In some embodiments, the syringe positioning assembly may further include a blocking assembly 140. The blocking assembly 140 may include a blocking member 141. The blocking member 141 is connected to the housing 100 and located outside the housing 100. The blocking member 141 is located on a side of the handle 210 away from the carrying face 110, and the blocking member 141 is configured to limit an opening angle of the handle 210. The blocking member 141 is located on a rotation trajectory of the handle 210 and can provide a limiting function to prevent the handle 210 from continuing to rotate in the direction away from the carrying face 110, so as to limit the maximum angle by which the handle 210 can rotate. The maximum angle by which the handle 210 can rotate can be adjusted by adjusting the height of the blocking member 141.

In some embodiments, the blocking assembly 140 may further include a sealing member 142. The sealing member 142 may be located in the housing 100 and abut against the housing 100. The sealing member 142 may be sleeved on the first connecting shaft 220. By providing the sealing member 142, it is possible to reduce the entry of external impurities and the like into the inner cavity 130 through a hole structure of the housing 100 for the first connecting shaft 220 to pass through, so as to prolong the service life of the structural members in the inner cavity 130. As an example, the sealing member 142 may be connected to the blocking member 141. For example, the sealing member 142 and the blocking member 141 may also be one-piece structural members, both made of silicone or other materials. This is not limited in the present application.

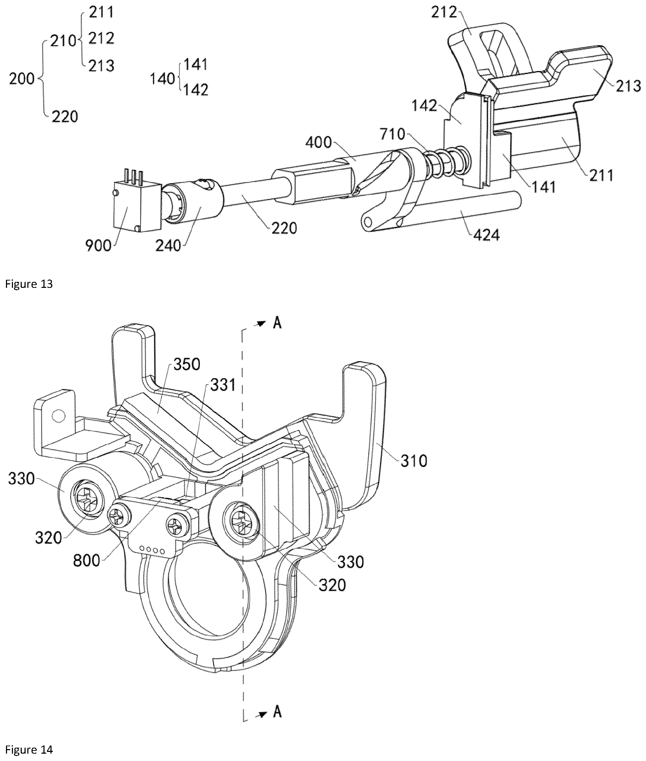

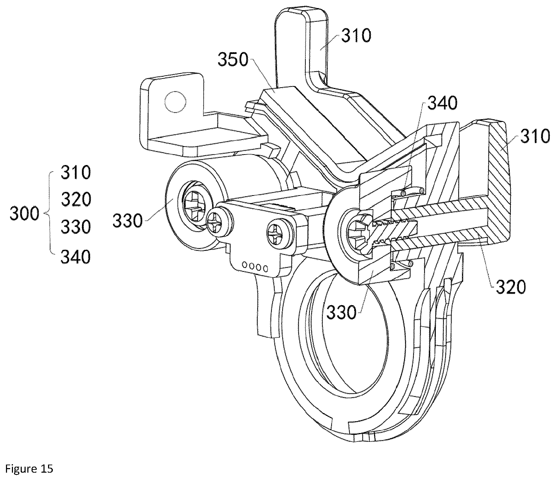

As shown in FIGS. 12, 14 and 15, FIG. 14 is a partial schematic structural diagram of the syringe positioning assembly shown in FIG. 12. FIG. 15 is a schematic cross-sectional structural diagram of the syringe positioning assembly shown in FIG. 14 along A-A.

In some embodiments, the syringe positioning assembly may further include a fixing member 300. The fixing member 300 may be movably connected to the housing 100. The fixing member 300 may include a fixing piece 310. The fixing piece 310 may be located outside the housing 100 and opposite to the fixing face 120, and the rolled piece of the syringe may be fixed between the fixing piece 310 and the fixing face 120 of the housing 100. Specifically, the fixing member 300 can move relative to the housing 100 such that the distance between the fixing piece 310 and the fixing face 120 can be adjusted. As an example, the fixing member 300 may move away from the fixing face 120 to increase the distance between the fixing piece 310 and the fixing face 120. When the distance between the fixing piece 310 and the fixing face 120 increases to be greater than or equal to the thickness of the flange of the syringe, the flange of the syringe can be smoothly placed between the fixing piece 310 and the fixing face 120 of the housing 100. Alternatively, the fixing member 300 may move toward the fixing face 120 to decrease the distance between the fixing piece 310 and the fixing face 120. When the flange of the syringe is located between the fixing piece 310 and the fixing face 120, the movement of the fixing member 300 toward the fixing face 120 can implement the process of clamping the flange of the syringe, thereby achieving the purpose of fixing the flange of the barrel of the syringe.

It can be understood that, for the convenience of description below, the syringe positioning assembly is defined to have a first direction X1, a second direction X2, a third direction A1 and a fourth direction A2. The direction in which the fixing piece 310 moves away from the fixing face 120 of the housing 100 is defined as the first direction X1, the direction in which the fixing piece 310 moves toward the fixing face 120 of the housing 100 is defined as the second direction X2, the direction in which the handle 210 rotates away from the carrying face 110 of the housing 100 is defined as the third direction A1, and the direction in which the handle 210 rotates toward the carrying face 110 of the housing 100 is defined as the fourth direction A2. In other implementations, the coordinate system setting of the syringe positioning assembly may be flexibly set according to specific actual requirements.

In some embodiments, the fixing member 300 may further include a second connecting shaft 320. One end of the second connecting shaft 320 is connected to the fixing piece 310, and the other end of the second connecting shaft 320 is movably arranged in the housing 100. As an example, the fixing member 300 may further include a stop block 330. The stop block 330 may be fixed to an end portion of the second connecting shaft 320, and the stop block 330 is located in the housing 100. The projection of the stop block 330 on the housing 100 may cover the diameter of the pin hole in the housing 100 for the second connecting shaft 320 to pass through. The stop block 330 is configured to limit the maximum distance by which the second connecting shaft 320 moves in the first direction X1, to prevent the fixing member 300 from moving excessively in the first direction X1, moving out of the housing 100, and being separated from the housing 100, which otherwise affects the reliability of the syringe positioning assembly. In the embodiment of the present application, an example is taken for description, in which the stop block 330 is fixedly connected to the second connecting shaft 320 via screws. In other embodiments, the stop block 330 may alternatively be fixed to the end portion of the second connecting shaft 320 by welding, gluing or other means. This is not limited in the present application.

In some embodiments, the fixing member 300 may further include an elastic ring 340. The elastic ring 340 is sleeved on the second connecting shaft 320. One end of the elastic ring 340 is connected to the housing 100, and the other end of the elastic ring 340 abuts against the stop block 330. The elastic ring 340 can push against and move the stop block 330 in the axial direction of the second connecting shaft 320, so as to drive the fixing piece 310 to move toward the fixing face 120. When the fixing member 300 moves away from the fixing face 120 of the housing 100, that is, when the fixing member 300 moves in the first direction X1, the elastic ring 340 may be pressed by the stop block 330 and the housing 100 and deformed. In this way, when the stop block 330 is no longer moved in the first direction X1 by the acting force in the first direction X1, a deformation restoring force of the elastic ring 340 can drive the stop block 330 to move in the second direction X2, that is, the fixing piece 310 moves toward the fixing face 120 of the housing 100, so that the fixing member 300 can clamp the flange of the syringe. Therefore, when the distance between the fixing piece 310 and the fixing face 120 of the housing 100 increases such that the flange of the syringe can be placed between the fixing member 300 and the fixing face 120 of the housing 100, the user only needs to cancel the acting force applied to the fixing member 300 in the first direction X1 in order to enable the fixing member 300 to automatically move in the second direction X2 to clamp the flange of the syringe, thereby effectively simplifying the operation of the syringe positioning assembly of the present application.

In some embodiments, there may be two second connecting shafts 320. The two second connecting shafts 320 are connected at intervals to the fixing piece 310. There also may be two elastic rings 340 and two stop blocks 330. The two second connecting shafts 320, the two elastic rings 340 and the two stop blocks 330 are arranged in one-to-one correspondence. By providing that there may be two elastic rings 340, after the acting force applied to the fixing piece 310 in the first direction X1 is canceled, two sides of the fixing piece 310 can be subjected to elastic restoring forces of the elastic rings 340, so that the acting forces applied on both sides of the fixing piece 310 can be balanced, improving the stability of the posture of the fixing piece 310 when moving in the second direction X2, and avoiding phenomena such as tilting of the fixing piece 310 when force is applied to one side thereof.

In some embodiments, the syringe positioning assembly may further include a mounting block 350. The stop block 330 and the fixing piece 310 are located on two opposite sides of the mounting block 350. The second connecting shaft 320 passes through the mounting block 350. The mounting block 350 may be fixed in the housing 100 by snap-fitting or other means, to mount the fixing member 300 to the housing 100. In other embodiments, the syringe positioning assembly may not include the mounting block 350. This is not limited in the present application. End portions of the mounting block 350 and the fixing piece 310 that face the syringe may be V-shaped, arc-shaped, or a combination of various shapes, so as to avoid other structures of the syringe such as the barrel and adapt to the shape thereof when the syringe is fixed to the syringe positioning assembly.

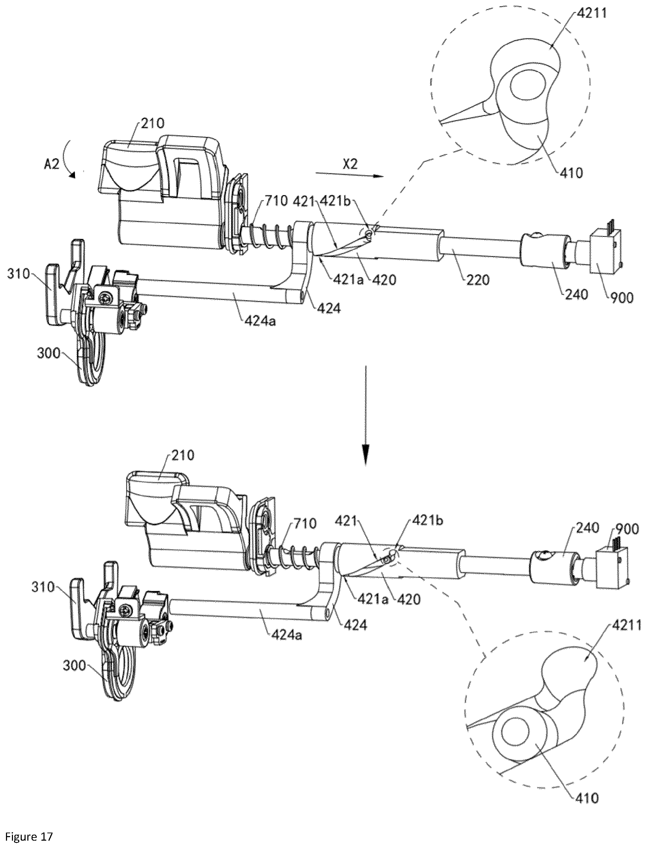

As shown in FIGS. 12, 16 and 17, FIG. 16 is a schematic diagram of a working process of a syringe positioning assembly provided by an embodiment of the present application. FIG. 17 is a schematic diagram of another working process of a syringe positioning assembly provided by an embodiment of the present application.

In some embodiments, the syringe positioning assembly may include a transmission member 400. The transmission member 400 may be connected to the first connecting shaft 220 and the fixing member 300. When the handle 210 rotates away from the carrying face 110 (as shown in FIG. 11), that is, when the handle 210 rotates in the third direction A1, the rotating member 200 can drive the fixing piece 310 to move away from the fixing face 120 by means of the transmission member 400. In the embodiment of the present application, by providing the transmission member 400, the movement processes of the rotating member 200 and the fixing member 300 can be coupled, so that when the handle 210 rotates away from the carrying face 110, that is, when the handle 210 moves in the third direction A1, the fixing piece 310 of the fixing member 300 can also be subjected to an acting force and move away from the fixing face 120 along with the rotation of the rotating member 200, that is, the fixing piece 310 can be driven to move in the first direction X1. In this way, the movement processes of the handle 210 and the fixing piece 310 can be controlled just by operating the handle 210, to implement the process of fixing the barrel of the syringe and the flange of the syringe. Compared with the prior art, in which it is necessary to operate corresponding independent components respectively to fix the barrel of the syringe and the flange of the syringe, the syringe positioning assembly of the present application can effectively improve the working efficiency of fixing the syringe.

Specifically, in some embodiments, the transmission member 400 may include a first transmission portion 410 and a second transmission portion 420 which mate with each other. The first transmission portion 410 is fixed to the first connecting shaft 220, and the second transmission portion 420 is axially movably sleeved on the first connecting shaft 220. The second transmission portion 420 is provided with a guide groove 421. The guide groove 421 may be of a spiral structure, and the first transmission portion 410 is embedded in the guide groove 421. The first transmission portion 410 can rotate along with the rotation of the first connecting shaft 220. When the first transmission portion 410 is embedded in the guide groove 421 of the second transmission portion 420, the guide groove 421 can function to limit the first transmission portion 410, and the rotational force of the first transmission portion 410 can act on an inner wall of the guide groove 421 so as to drive the second transmission portion 420 to move axially.

As an example, the groove profile of the guide groove 421 may mate with the first transmission portion 410. The guide groove 421 has a first end 421a and a second end 421b. The first end 421a is closer to the fixing piece 310 than the second end 421b. After the first transmission portion 410 rotates in the third direction A1, the first transmission portion 410 can also move relative to the second transmission portion 420 in a direction from the first end 421a to the second end 421b of the guide groove 421. In other words, after the first transmission portion 410 rotates in the third direction A1, the second transmission portion 420 can be pushed to move axially relative to the first connecting shaft 220 to convert a rotational force into an acting force for linear motion. Since the second transmission portion 420 is detachably connected to the fixing member 300, when the second transmission portion 420 moves, it can drive the fixing member 300 to move in the first direction X1, thereby realizing the transmission function of the transmission member 400 and implementing the process of fixing the barrel of the syringe and the flange of the syringe by rotating the handle 210.

The third direction A1 of rotation of the rotating member 200 may be arranged around the first direction X1 of movement of the fixing member 300, that is, the first direction X1 is the axis of a rotation trajectory of the rotating member 200, and the guide groove 421 may be arranged to be arranged spirally around the first direction X1 on the second transmission portion 420, so that the guide groove 421 can not only be distributed in the first direction X1, but the guide groove 421 can also be distributed in the third direction A1. Therefore, during the movement of the first transmission portion 410 relative to the second transmission portion 420 along the guide groove 421, the rotational force of the first transmission portion 410 can act on an inner wall of the guide groove 421, such that the rotational force of the first transmission portion 410 can be decomposed into a linear driving force acting on the second transmission portion 420 in the first direction X1, the second transmission portion 420 thus moves relative to the first transmission portion 410 in the first direction X1, and finally the fixing member 300 moves in the direction away from the fixing face 120 of the housing 100.

In some embodiments, the second end 421b may be provided with a positioning groove 4211. The positioning groove 4211 is configured to maintain the handle 210 in the maximum position when the first transmission portion 410 is located in the positioning groove 4211. As an example, the positioning groove 4211 may be formed in a direction perpendicular to an axis of rotation of the first connecting shaft 220. By providing the positioning groove 4211, when the first transmission portion 410 is located in the positioning groove 4211, if no additional force is applied to cause the first transmission portion 410 to slide out of the positioning groove 4211, the first transmission portion 410 is limited in the positioning groove 4211, thereby preventing the rotating member 200 from continuing to rotate, so that the handle 210 can be in a hovering state relative to the carrying face 110 (as shown in FIG. 11), and the user's hands can be released. By providing the positioning groove 4211 at the second end 421b, the handle 210 can be in the hovering state when having the largest opening angle relative to the carrying face 110. At this time, the space between the handle 210 and the carrying face 110 is the largest, which can facilitate the smooth placement of the syringe on the carrying face 110.

In some embodiments, the second transmission portion 420 is further provided with a push member 424. The push member 424 can move axially and is configured to push against the fixing member 300 such that the fixing piece 310 moves away from the fixing face 120 (as shown in FIG. 12). The transmission member 400 can be in contact with the fixing member 300 by means of the push member 424, to implement the process of pushing the fixing member 300. As an example, the push member 424 may include an abutment portion 424a. The abutment portion 424a may be parallel to the first connecting shaft 220, and the abutment portion 424a is configured to push against the fixing member 300. In the process of the transmission member 400 driving the push member 424 to move such that the push member is in contact with the fixing member 300 and pushes the fixing member 300, by setting the abutment portion 424a parallel to the first connecting shaft 220, it can be ensured that the transmission member 400 can provide the fixing member 300 with an acting force in the first direction X1 to push the fixing member 300 to move in the first direction X1, to implement the process of fixing the flange of the syringe. Specifically, the abutment portion 424a may be in contact with one of the stop blocks 330 of the fixing member 300 to push the fixing member 300 to move in the first direction X1. In other embodiments, the abutment portion 424a may also be in contact with other components of the fixing member 300 to implement the pushing process. This is not limited in the present application.

In some embodiments, the second transmission portion 420 may be provided with a first limiting structure 425 (as shown in FIG. 12), and a second limiting structure 150 may be provided in the housing 100 (as shown in FIG. 12). The second limiting structure 150 may mate with the first limiting structure 425 to implement the process of limiting the rotation of the second transmission portion 420. As an example, the second limiting structure 150 may include two opposite bumps (as shown in FIG. 12) arranged spaced apart from each other, the first limiting structure 425 may be a channel provided in the second transmission portion 420, and the first limiting structure 425 may be located between the two bumps. By arranging the first limiting structure 425 between the two bumps, the second limiting structure 150 can limit the second transmission portion 420 in the rotation direction of the rotating member 200, so as to limit the movement trajectory of the second transmission portion 420 to prevent the second transmission portion 420 from rotating relative to the housing 100 as the rotating member 200 rotates, which otherwise causes a component such as the push member 424 connected to the second transmission portion 420 to rotate in the inner cavity 130 and collide with other structural members in the inner cavity 130, thus affecting the service life of the syringe positioning assembly. The first limiting structure 425 may also be a groove or a protrusion provided on the second transmission portion 420. This is not limited in the present application.

In some embodiments, the syringe positioning assembly further includes a compression spring 710. The compression spring 710 may be sleeved on the first connecting shaft 220. The compression spring 710 has one end abutting against the sealing member 142, and the other end abutting against the second transmission portion 420. A deformation restoring force of the compression spring 710 can drive the second transmission portion 420 to move axially, so as to drive the handle 210 to rotate toward the carrying face 110 (as shown in FIG. 11). When the rotating member 200 rotates away from the carrying face 110, that is, when the handle 210 rotates in the third direction A1, the second transmission portion 420 moves in the first direction X1 to push the fixing piece 310 to move in the first direction X1. During the movement of the second transmission portion 420 in the first direction X1, the compression spring 710 is pressed by the second transmission portion 420 and deformed. In this way, when the handle 210 is no longer rotated by the acting force, that is, when the second transmission portion 420 no longer presses the compression spring 710 in the first direction X1, the deformation restoring force of the compression spring 710 can drive the second transmission portion 420 to move in the second direction X2, so that the mating between the second transmission portion 420 and the first transmission portion 410 can convert the acting force of the compression spring 710 in the second direction X2 into an acting force for driving the rotating member 200 to rotate in the fourth direction A2, and the rotation of the handle 210 in the fourth direction A2 causes the distance between the handle 210 and the carrying face 110 to decrease. Then, after the barrel of the syringe is placed between the handle 210 and the carrying face 110, it is only necessary to cancel the acting force applied to the handle 210 in the third direction A1 in order to enable the handle 210 to be automatically moved in the fourth direction A2 to clamp the barrel of the syringe, so that the process of clamping the barrel of the syringe by the handle 210 is effectively simplified, and the adaptive process of clamping barrels of syringes of different sizes by the syringe positioning assembly is implemented, making it convenient for the user to use.

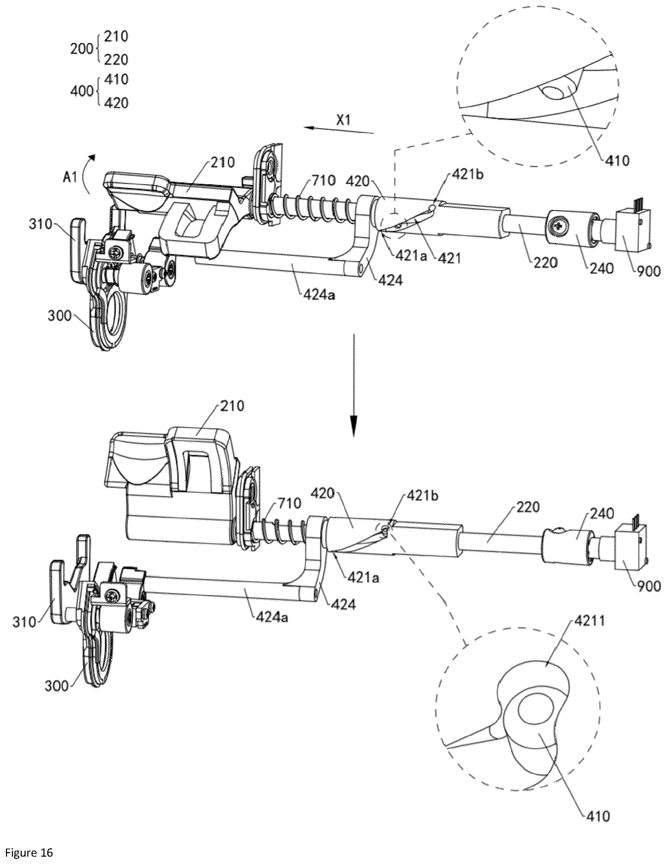

Referring to FIGS. 16 and 17 again, the process of fixing the syringe by the syringe positioning assembly may generally include a lifting process, a hovering process and a clamping process. In other embodiments, the process of fixing the syringe by the syringe may not include the hovering process, or may include more processes. This is not limited in the present application.

The lifting process may generally lie in that the handle 210 rotates in the third direction A1 to release the space between the handle 210 and the carrying face 110. At this time, the first transmission portion 410 moves from the first end 421a toward the second end 421b, and the second transmission portion 420 moves toward the side close to the fixing member 300 until the first transmission portion 410 moves to the second end 421b, which is deemed to be the end of the lifting process.

The hovering process may generally lie in that the first transmission portion 410 is located in the positioning groove 4211 at the second end 421b, and the positioning groove 4211 limits the movement of the first transmission portion 410. At this time, the space between the handle 210 and the carrying face 110 is the largest, facilitating the smooth mounting of the syringe into the syringe positioning assembly. In the embodiments of the present application, the position of the handle 210 relative to the carrying face 110 at this time is also regarded as the maximum position of the handle 210. The handle 210 is maintained at the maximum position relative to the carrying face 110. After the syringe is mounted, the user gently shifts the handle 210 in the fourth direction A2 such that the first transmission portion 410 is separated from the positioning groove 4211, which is deemed to be the end of the hovering process.

The clamping process may generally lie in that the compression spring 710 provides a deformation restoring force to push the second transmission portion 420 to move in the second direction X2, such that the first transmission portion 410 moves from the second end 421b toward the first end 421a, so as to drive the first connecting shaft 220 to rotate in the fourth direction A2, so that the handle 210 rotates in the fourth direction A2, and the space between the handle 210 and the carrying face 110 decreases until the handle 210 comes into contact with the barrel of the syringe, which is deemed to be the end of the clamping process. In this case, during the movement of the second transmission portion 420 in the second direction X2, the distance between the abutment portion 424a and the stop block 330 increases until they are separated. During this process, after the acting force applied to the fixing member 300 by the abutment portion 424a in the first direction X1 is withdrawn, the fixing piece 310 also moves in the second direction X2 under the pushing of the deformation restoring force of the elastic ring 340, to implement the process of clamping the rolled piece of the syringe.

In the embodiments of the present application, with the mating of the structural members in the syringe positioning assembly during different working processes, the process of clamping the barrel and the flange of the syringe can be implemented, and the efficiency of fixing the syringe by the syringe positioning assembly can be effectively improved.

Referring to FIGS. 12 and 14 together, in some implementations, the fixing member 300 may further include a paddle 331, and the syringe positioning assembly may further include a trigger switch 800. The trigger switch 800 is connected to the housing 100, the paddle 331 is connected to the second connecting shaft 320, and the paddle 331 is configured to move along with the second connecting shaft 320 so as to be in contact with or separated from the trigger switch 800. As an example, in the present application, the paddle 331 may be fixed on the stop block 330 and move along with the second connecting shaft 320 together with the stop block 330. In other embodiments, the paddle 331 may alternatively be directly connected to an end portion of the second connecting shaft 320. This is not limited in the present application.

During the movement of the fixing piece 310, the paddle 331 can move in the first direction X1 or the second direction X2 along with the second connecting shaft 320, so as to be in contact with or separated from the trigger switch 800. When the distance between the fixing piece 310 of the fixing member 300 and the fixing face 120 of the housing 100 is minimal, the paddle 331 is configured to be capable of acting on the trigger switch 800 such that the trigger switch 800 is turned on to generate a corresponding signal. When the paddle 331 moves in the first direction X1 along with the second connecting shaft 320, the paddle 331 is separated from the trigger switch 800, the trigger switch 800 is turned off, and the corresponding signal disappears. When the flange of the barrel of the syringe is clamped between the fixing piece 310 and the housing 100, the flange of the syringe blocks the contact between the paddle 331 and the trigger switch 800, and the paddle 331 thus remains separated from the trigger switch 800. Therefore, when the trigger switch 800 continues to have no signal, it can be determined that the flange of the barrel of the syringe has been mounted between the fixing piece 310 and the fixing face 120 of the housing 100, thereby facilitating the user to accurately determine the mounting state of the syringe.

In some embodiments, the syringe positioning assembly may further include a coupling 240 and an angle sensor 900. The angle sensor 900 may be fixed to the inner wall of the housing 100. The coupling 240 is located in the inner cavity 130 and is sleeved on the first connecting shaft 220, and the angle sensor 900 is connected to an end portion of the first connecting shaft 220 via the coupling 240. As an example, the angle sensor 900 may be a rotary potentiometer. By providing the angle sensor 900, the user can also obtain a rotation angle of the first connecting shaft 220, then obtain a movement stroke of the fixing member 300, and finally obtain a distance between the fixing piece 310 and the fixing face 120 of the housing 100, to facilitate the user to determine whether the flange of the barrel of the syringe can be placed between the fixing piece 310 and the fixing face 120 of the housing 100.

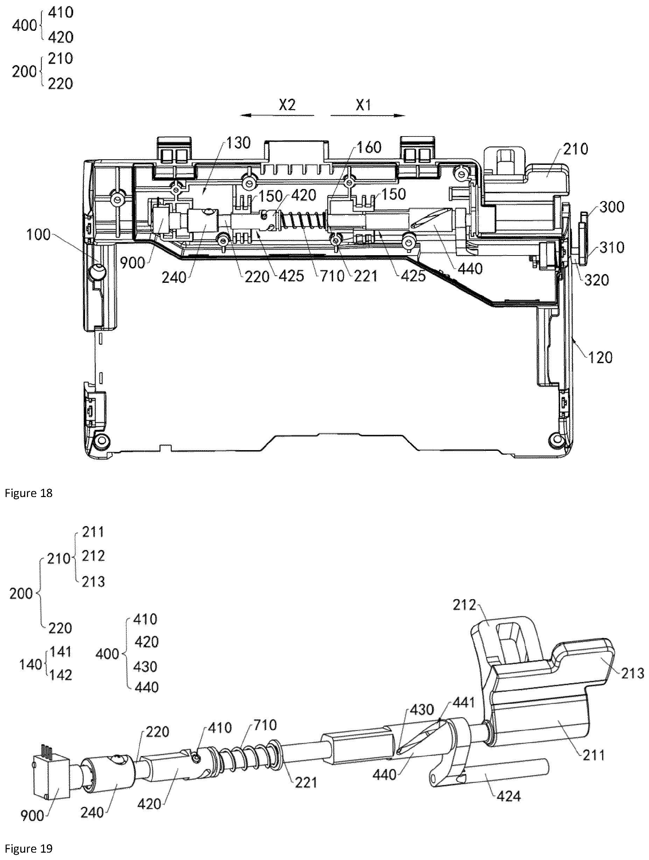

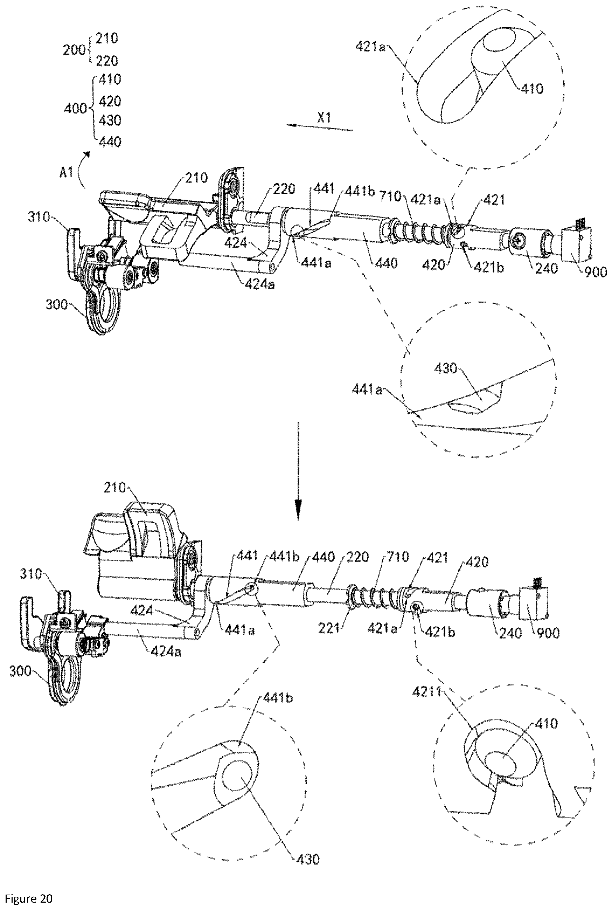

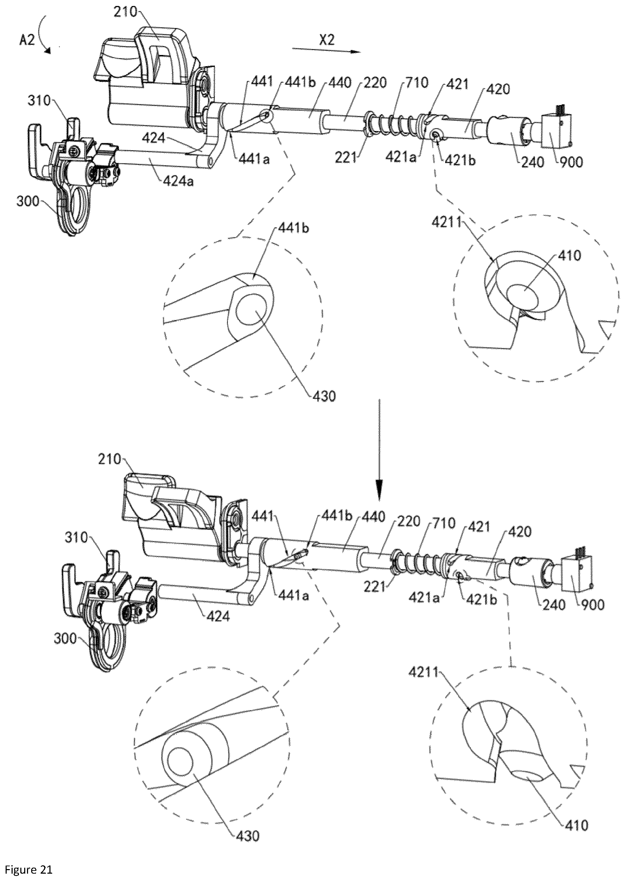

In the above embodiments, taking the push member 424 being arranged on the second transmission portion 420 as an example, the syringe positioning assembly is designed accordingly. In other embodiments, the push member 424 and the second transmission portion 420 may alternatively have other relative positional relationships. For example:
As shown in FIGS. 18, 19, 20 and 21, FIG. 18 is a schematic structural diagram of another syringe positioning assembly provided by the present application. FIG. 19 is a partial schematic structural diagram of the syringe positioning assembly shown in FIG. 18. FIG. 20 is a schematic diagram of a working process of the syringe positioning assembly shown in FIG. 19. FIG. 21 is a schematic diagram of another working process of the syringe positioning assembly shown in FIG. 19. This embodiment may include most of the technical solutions of the above embodiments. The following mainly describes the differences between the two, and most of the same contents between the two will not be described again.

In some embodiments, the transmission member 400 may further include a third transmission portion 430 and a fourth transmission portion 440 which mate with each other. The third transmission portion 430 is fixed to the first connecting shaft 220, the fourth transmission portion 440 is axially movably sleeved on the first connecting shaft 220, the fourth transmission portion 440 may be provided with a limiting groove 441 and a push member 424, the limiting groove 441 is of a spiral structure, and the third transmission portion 430 is embedded in the limiting groove 441. The push member 424 can move axially and is configured to push against the fixing member 300 such that the fixing piece 310 moves away from the fixing face 120. The fourth transmission portion 440 is arranged spaced apart from the second transmission portion 420, and the compression spring 710 may be located between the fourth transmission portion 440 and the second transmission portion 420.

When the rotating member 200 rotates away from the carrying face 110 (as shown in FIG. 11), that is, when the handle 210 rotates in the third direction A1, the third transmission portion 430 rotates along with the rotating member 200 to push the fourth transmission portion 440 to move in the first direction X1, so as to drive the push member 424 to push the fixing piece 310 to move in the first direction X1, thereby implementing the process of coupling the movements of the rotating member 200 and the fixing member 300 by means of the transmission member 400. In this case, the first transmission portion 410 also rotates along with the rotating member 200, to push the second transmission portion 420 to move in the first direction X1. During the movement of the second transmission portion 420 in the first direction X1, the compression spring 710 is pressed by the second transmission portion 420 and deformed. In this way, when the handle 210 is no longer rotated by the acting force, that is, when the second transmission portion 420 no longer presses the compression spring 710 in the first direction X1, the deformation restoring force of the compression spring 710 can drive the second transmission portion 420 to move in the second direction X2, so that the mating between the second transmission portion 420 and the first transmission portion 410 can convert the acting force of the compression spring 710 in the second direction X2 into an acting force for driving the rotating member 200 to rotate in the fourth direction A2, and the rotation of the handle 210 in the fourth direction A2 causes the distance between the handle 210 and the carrying face 110 to decrease. Then, after the barrel of the syringe is placed between the handle 210 and the carrying face 110, it is only necessary to cancel the acting force applied to the handle 210 in the third direction A1 in order to enable the handle 210 to be automatically moved in the fourth direction A2 to clamp the barrel of the syringe, so that the process of clamping the barrel of the syringe by the handle 210 is effectively simplified, and the adaptive process of clamping barrels of different sizes by the syringe positioning assembly is implemented, making it convenient for the user to use.

In some embodiments, the limiting groove 441 has a third end 441a and a fourth end 441b. The third end 441a is closer to the fixing piece 310 than the fourth end 441b. When the handle 210 rotates to a maximum position, the first transmission portion 410 is located at the second end 421b, the third transmission portion 430 is located at the fourth end 441b, and the push member 424 moves the fixing piece 310 to a maximum displacement. In other words, the limiting groove 441 and the guide groove 421 extend in the same direction, and both may be configured to be arranged spirally around the first direction X1. It can be understood that, for example, when the handle 210 rotates in the third direction A1, the movement directions of the first transmission portion 410 and the third transmission portion 430 should be the same. If the limiting groove 441 and the guide groove 421 extend in opposite directions, the guide groove 421 will block the rotation process of the first transmission portion 410, or the limiting groove 441 will block the rotation process of the third transmission portion 430, affecting the rotation process of the rotating member 200, thereby affecting the working process of the syringe positioning assembly.

In some embodiments, in the axial direction of the first connecting shaft 220, the distance between the two ends of the limiting groove 441 is greater than the distance between the two ends of the guide groove 421. That is, when the handle 210 rotates by the same angle, the movement path of the fourth transmission portion 440 relative to the housing 100 in the axial direction of the first connecting shaft 220 is longer than the movement path of the second transmission portion 420 relative to the housing 100 in the axial direction of the first connecting shaft 220, facilitating the reduction of the required length of the push member 424.

In some embodiments, a positioning member 221 is provided on the first connecting shaft 220, the positioning member 221 is located between the fourth transmission portion 440 and the second transmission portion 420, and the two ends of the compression spring 710 respectively abut against the positioning member 221 and the second transmission portion 420. A stopping member 160 is provided in the housing 100, the positioning member 221 is located between the stopping member 160 and the compression spring 710, and the stopping member 160 is configured to axially limit the positioning member 221. With the mating between the stopping member 160 and the positioning member 221, the acting direction of the deformation restoring force of the compression spring 710 can be limited, ensuring that one end of the compression spring 710 is fixed to the first connecting shaft 220, and preventing the compression spring 710 from slipping on the first connecting shaft 220, which otherwise affects the effect of its deformation restoring force on the second transmission portion 420.

In some embodiments, the second transmission portion 420 and the fourth transmission portion 440 are each provided with a first limiting structure 425, and a second limiting structure 150 is provided in the housing 100. The second limiting structure 150 mates with the first limiting structures 425 to limit the rotation of the second transmission portion 420 and the fourth transmission portion 440. As an example, the second limiting structure 150 may include two opposite bumps (as shown in FIG. 18) arranged spaced apart from each other, the first limiting structure 425 may be a channel provided on the second transmission portion 420 and a channel provided on the fourth transmission portion 440, and the first limiting structure 425 may be located between the two bumps. By arranging the first limiting structure 425 between the two bumps, the second limiting structure 150 can limit the second transmission portion 420 and the fourth transmission portion 440 in the rotation direction of the rotating member 200, so as to limit the movement trajectory of the second transmission portion 420 and the fourth transmission portion 440 to prevent the second transmission portion 420 and the fourth transmission portion 440 from rotating relative to the housing 100 as the rotating member 200 rotates, which otherwise causes a component such as the push member 424 connected to the fourth transmission portion 440 to rotate in the inner cavity 130 and collide with other structural members in the inner cavity 130, thus affecting the service life of the syringe positioning assembly. The first limiting structure 425 may also be a structure such as a protrusion or a groove provided on the second transmission portion 420 or the fourth transmission portion 440. This is not limited in the present application.

The process of fixing the syringe by the syringe positioning assembly may generally include a lifting process, a hovering process and a clamping process. In other embodiments, the process of fixing the syringe by the syringe may not include the hovering process, or may include more processes. This is not limited in the present application.

The lifting process may generally lie in that the handle 210 rotates in the third direction A1 to release the space between the handle 210 and the carrying face 110 (as shown in FIG. 11). At this time, the first transmission portion 410 moves from the first end 421a toward the second end 421b, the third transmission portion 430 moves from the third end 441a toward the fourth end 441b, and the push member 424 moves toward the side close to the fixing member 300 until the first transmission portion 410 moves to the second end 421b or/and the third transmission portion 430 moves to the fourth end 441b, which is deemed to be the end of the lifting process.

The hovering process may generally lie in that the first transmission portion 410 is located in the positioning groove 4211 at the second end 421b, and the positioning groove 4211 can limit the movement of the first transmission portion 410. At this time, the space between the handle 210 and the carrying face 110 is the largest, facilitating the smooth mounting of the syringe into the syringe positioning assembly. The position of the handle 210 relative to the carrying face 110 at this time is regarded as the maximum position of the handle 210. The handle 210 is maintained at the maximum position relative to the carrying face 110. After the syringe is mounted, the user can gently shift the handle 210 in the fourth direction A2 such that the first transmission portion 410 is separated from the positioning groove 4211, which is deemed to be the end of the hovering process.

The clamping process may generally lie in that the compression spring 710 provides a deformation restoring force to push the second transmission portion 420 to move in the second direction X2 and act on the first transmission portion 410, such that the first transmission portion 410 moves from the second end 421b toward the first end 421a, so as to drive the first connecting shaft 220 to rotate in the fourth direction A2, so that the handle 210 rotates in the fourth direction A2, and the space between the handle 210 and the carrying face 110 decreases until the handle 210 comes into contact with the barrel of the syringe, which is deemed to be the end of the clamping process. In this case, during the rotation of the handle 210 in the fourth direction A2, the fourth transmission portion 440 also moves in the first direction X1, so that the distance between the abutment portion 424a and the stop block 330 increases until they are separated. The acting force applied to the fixing member 300 by the abutment portion 424a in the first direction X1 is withdrawn, and the fixing piece 310 also moves in the second direction X2 under the action of the deformation restoring force of the elastic ring 340, to implement the process of clamping the rolled piece of the syringe.

In the embodiments of the present application, with the mating of the structural members in the syringe positioning assembly during different working processes, the process of clamping the barrel and the flange of the syringe can be implemented, and the efficiency of fixing the syringe by the syringe positioning assembly can be effectively improved.

Based on the above syringe positioning assembly, the embodiments of the present application also provide a syringe pump, which includes the above syringe positioning assembly.

It should be finally noted that the foregoing implementations are merely used for illustrating rather than limiting the technical solutions of the present application. Although the present application has been illustrated in detail with reference to the foregoing implementations, it should be understood by those of ordinary skill in the art that the technical solutions specified in the foregoing implementations could still be modified, or some or all of the technical features thereof could be equivalently substituted; and these modifications or substitutions do not make the essence of the corresponding technical solution depart from the scope of the technical solutions of the implementations of the present application.

## Claims

1. A syringe positioning assembly, comprising:
a housing (100) having a carrying face (110) and a fixing face (120);
a rotating member (200) rotatably arranged on the housing (100), the rotating member (200) being configured to be rotatable in a direction toward or away from the carrying face (110);
a fixing member (300) movably connected to the housing (100), the fixing member (300) being opposite to the fixing face (120); and
a transmission member (400) connected to the rotating member (200) and the fixing member (300);
wherein when the rotating member (200) rotates away from the carrying face (110), the rotating member (200) drives the fixing member (300) to move away from the fixing face (120) by means of the transmission member (400).

2. The syringe positioning assembly according to claim 1, wherein the rotating member (200) comprises a handle (210) and a first connecting shaft (220) which are connected to each other, wherein the first connecting shaft (220) is rotatably arranged in the housing (100), the handle (210) is located outside the housing (100), and the handle (210) is configured to clamp a syringe at the carrying face (110);
the fixing member (300) comprises a fixing piece (310) located outside the housing (100) and opposite to the fixing face (120);
the transmission member (400) is connected to the first connecting shaft (220) and the fixing member (300), respectively; when the handle (210) rotates away from the carrying face (110), the rotating member (200) drives the fixing piece (310) to move away from the fixing face (120) by means of the transmission member (400); and
the syringe positioning assembly further comprises a compression spring (710), wherein the compression spring (710) is sleeved on the first connecting shaft (220); one end of the compression spring (710) is connected to the first connecting shaft (220), and the other end of the compression spring (710) abuts against the transmission member (400); and the compression spring (710) is capable of pushing against and axially moving the transmission member (400).

3. The syringe positioning assembly according to claim 2, wherein the transmission member (400) comprises a first transmission portion (410) and a second transmission portion (420) which mate with each other, wherein the first transmission portion (410) is fixed to the first connecting shaft (220), the second transmission portion (420) is axially movably sleeved on the first connecting shaft (220), the second transmission portion (420) is provided with a guide groove (421) of a spiral structure, and the first transmission portion (410) is embedded in the guide groove (421).

4. The syringe positioning assembly according to claim 3, wherein the second transmission portion (420) is further provided with a push member (424), which is axially movable and configured to push against the fixing member (300) such that the fixing piece (310) moves away from the fixing face (120).

5. The syringe positioning assembly according to claim 3, wherein the transmission member (400) further comprises a third transmission portion (430) and a fourth transmission portion (440) which mate with each other, wherein the third transmission portion (430) is fixed to the first connecting shaft (220), the fourth transmission portion (440) is axially movably sleeved on the first connecting shaft (220), the fourth transmission portion (440) is provided with a limiting groove (441) and a push member (424), the limiting groove (441) is of a spiral structure, and the third transmission portion (430) is embedded in the limiting groove (441); the push member (424) is axially movable and configured to push against the fixing member (300) such that the fixing piece (310) moves away from the fixing face (120); and
the fourth transmission portion (440) is arranged spaced apart from the second transmission portion (420), and the compression spring (710) is located between the fourth transmission portion (440) and the second transmission portion (420).

6. The syringe positioning assembly according to claim 4 or 5, wherein the push member (424) comprises an abutment portion (424a), wherein the abutment portion (424a) is parallel to the first connecting shaft (220), and the abutment portion (424a) is configured to push against the fixing member (300).

7. The syringe positioning assembly according to claim 5, wherein in an axial direction of the first connecting shaft (220), a distance between two ends of the limiting groove (441) is greater than a distance between two ends of the guide groove (421).

8. The syringe positioning assembly according to claim 5, wherein the guide groove (421) has a first end (421a) and a second end (421b), the first end (421a) being closer to the fixing piece (310) than the second end (421b); the limiting groove (441) has a third end (441a) and a fourth end (441b), the third end (441a) being closer to the fixing piece (310) than the fourth end (441b); and
when the handle (210) rotates to a maximum position, the first transmission portion (410) is located at the second end (421b), the third transmission portion (430) is located at the fourth end (441b), and the push member (424) moves the fixing piece (310) to a maximum displacement.

9. The syringe positioning assembly according to claim 8, wherein the second end (421b) is provided with a positioning groove (4211); and the positioning groove (4211) is configured to maintain the handle (210) in the maximum position when the first transmission portion (410) is located in the positioning groove (4211).

10. The syringe positioning assembly according to claim 9, wherein a positioning member (221) is provided on the first connecting shaft (220), the positioning member (221) is located between the fourth transmission portion (440) and the second transmission portion (420), and the two ends of the compression spring (710) respectively abut against the positioning member (221) and the second transmission portion (420); and a stopping member (160) is provided in the housing (100), the positioning member (221) is located between the stopping member and the compression spring (710), and the stopping member (160) is configured to axially limit the positioning member (221).

11. The syringe positioning assembly according to claim 5, wherein the second transmission portion (420) and the fourth transmission portion (440) are each provided with a first limiting structure (425), a second limiting structure (150) is provided in the housing (100), the second limiting structure (150) mates with the first limiting structures (425) to limit the rotation of the second transmission portion (420) and the fourth transmission portion (440).

12. The syringe positioning assembly according to claim 2, further comprising a blocking member (141) connected to the housing (100) and located outside the housing (100), wherein the blocking member (141) is configured to limit an opening angle of the handle (210); and/or the syringe positioning assembly further comprises a blocking member (141) connected to the housing (100) and located outside the housing (100), wherein the blocking member (141) is configured to limit an opening angle of the handle (210); the syringe positioning assembly further comprises a sealing member (142) located in the housing (100) and abutting against the housing (100), wherein the sealing member (142) is sleeved on the first connecting shaft (220), and the sealing member (142) is connected to the blocking member (141); and/or the syringe positioning assembly further comprises a blocking member (141) connected to the housing (100) and located outside the housing (100), wherein the blocking member (141) is configured to limit an opening angle of the handle (210); the syringe positioning assembly further comprises a sealing member (142) located in the housing (100) and abutting against the housing (100), wherein the sealing member (142) is sleeved on the first connecting shaft (220), and the sealing member (142) is connected to the blocking member (141); the compression spring (710) is located between the sealing member (142) and the transmission member (400), and one end of the compression spring (710) abuts against the sealing member (142).

13. The syringe positioning assembly according to claim 2, wherein the fixing member (300) further comprises a second connecting shaft (320), one end of the second connecting shaft (320) is connected to the fixing piece (310), and the other end of the second connecting shaft (320) is movably arranged in the housing (100); and
the fixing member (300) further comprises a stop block (330) fixed to an end portion of the second connecting shaft (320) and located in the housing (100), the stop block (330) being configured to limit a maximum distance by which the second connecting shaft (320) moves in an axial direction of the second connecting shaft (320); and/or
the fixing member (300) further comprises a second connecting shaft (320), one end of the second connecting shaft (320) is connected to the fixing piece (310), and the other end of the second connecting shaft (320) is movably arranged in the housing (100); and
the fixing member (300) further comprises a stop block (330) fixed to an end portion of the second connecting shaft (320) and located in the housing (100), the stop block (330) being configured to limit a maximum distance by which the second connecting shaft (320) moves in an axial direction of the second connecting shaft (320); the fixing member (300) further comprises an elastic ring (340) sleeved on the second connecting shaft (320), wherein one end of the elastic ring (340) is connected to the housing (100), and the other end of the elastic ring (340) abuts against the stop block (330); and the elastic ring (340) is capable of pushing against and moving the stop block (330) in the axial direction of the second connecting shaft (320), so as to drive the fixing piece (310) to move toward the fixing face (120); and/or
the fixing member (300) further comprises a second connecting shaft (320), one end of the second connecting shaft (320) is connected to the fixing piece (310), and the other end of the second connecting shaft (320) is movably arranged in the housing (100); and
the fixing member (300) further comprises a stop block (330) fixed to an end portion of the second connecting shaft (320) and located in the housing (100), the stop block (330) being configured to limit a maximum distance by which the second connecting shaft (320) moves in an axial direction of the second connecting shaft (320); the fixing member (300) further comprises an elastic ring (340) sleeved on the second connecting shaft (320), wherein one end of the elastic ring (340) is connected to the housing (100), and the other end of the elastic ring (340) abuts against the stop block (330); and the elastic ring (340) is capable of pushing against and moving the stop block (330) in the axial direction of the second connecting shaft (320), so as to drive the fixing piece (310) to move toward the fixing face (120); there are two second connecting shafts (320), which are connected at intervals to the fixing piece (310), there are also two elastic rings (340) and two stop blocks (330), and the two second connecting shafts (320), the two elastic rings (340) and the two stop blocks (330) are arranged in one-to-one correspondence.

14. The syringe positioning assembly according to claim 13, wherein the fixing member (300) further comprises a paddle (331) and a trigger switch (800), wherein the trigger switch (800) is connected to the housing (100), the paddle (331) is connected to the second connecting shaft (320), and the paddle (331) is configured to move along with the second connecting shaft (320) so as to be in contact with or separated from the trigger switch (800).

15. A syringe pump, comprising a syringe positioning assembly of any one of claims 1-14.
